# EUROPEAN PATENT SPECIFICATION

(11) **EP 2 170 934 B1**
(45) Date of publication and mention of the grant of the patent: **21.01.2015**
(21) Application number: 08807203.8
(22) Date of filing: 13.06.2008
(51) Int. Cl.: C07K 14/08, A61K 38/16

(54) **CELL-PROLIFERATION INHIBITING VPG PROTEINS OR FRAGMENTS THEREOF**
VPG-PROTEINE ALS ZELLPROLIFERATIONSHEMMER ODER DEREN FRAGMENTE
PROTÉINES VPG INHIBANT LA PROLIFÉRATION CELLULAIRE OU FRAGMENTS CORRESPONDANTS

(30) Priority: 13.06.2007 EP 07290740
(43) Date of publication of application: 07.04.2010
(73) Proprietor: Centre National de la Recherche Scientifique, 75016 Paris (FR); Institute of Biochemistry and Biophysics - Polish Academy of Sciences, 02-106 Warszawa (PL); Université Joseph Fourier, 38041 Grenoble Cédex 9 (FR)
(72) Inventor: CHROBOCZEK, Jadwiga, F-38100 Grenoble (FR); ZAGORSKI, Wlodzimierz, PL-02665 Varsovie (PL); GRZELA, Renata, PL-25138 Kielce (PL)
(74) Representative: Rançon, Xavier Lucien Abel
(86) International application number: PCT/IB2008/002594
(87) International publication number: WO 2008/152527

(56) References cited:
- WO-A-01/40490
- US-A- 5 589 612
- DATABASE UNIPROTKB/TREMBL 1 November 1996 (1996-11-01), WELNICKI, M.A. AND BAULCOMBE, D.C.: "The nucleotide sequence of the central fragment of potato virus Y encoding 6K2 and vPg proteins" XP002450611 retrieved from EBI Database accession no. Q85258
- DATABASE EMBL/GENBANK/DDBJ 24 January 1994 (1994-01-24), WELNICKI, M.A. AND BAULCOMBE, D.C.: "Potato virus Y ; 6K2 gene" XP002450612 retrieved from EBI Database accession no. Z29526 cited in the application
- GRZELA R ET AL: "Potyvirus terminal protein VPg, effector of host eukaryotic initiation factor eIF4E" BIOCHIMIE (PARIS), vol. 88, no. 7, July 2006 (2006-07), pages 887-896, XP005595433 ISSN: 0300-9084 cited in the application

## Description

The present invention relates to the field of cell proliferation-inhibiting proteins. More particularly, the invention relates to a protein comprising or consisting of a VPg protein or fragments thereof that are cytotoxic to eukaryotic cells, and/or inhibit the cell proliferation. The invention also relates to medical uses of such a protein or fragments thereof.

Eukaryotic initiation factor eIF4E is a mRNA 5' cap-binding protein, which both attaches mRNA and binds the eIF4G component of the eIF4F initiation complex. A significant fraction of eIF4E (up to nearly 70%) localizes to the nucleus (Lejbkowicz *et al.,* 1992). It has been shown that nuclear eIF4E is involved in the nucleocytoplasmic transport of certain messenger RNAs (Lejbkowicz *et al.,* 1992; Rosenwald *et al.,* 1995; Rousseau *et al.,* 1996; Lai and Borden, 2000). It has been demonstrated that the unchecked over-expression of eIF4E can cause cell growth and malignant transformation, whereas reduction of the eIF4E level can reverse the transformed phenotype (reviewed in Sonenberg and Gingras, 1998). Furthermore, in humans, some tumor types exhibit elevated levels of eIF4E (Ruggero and Pandolfi, 2003). It appears that at high eIF4E levels there is increased cytoplasmic level of proteins involved in the malignant transformation, due to the increase in the eIF4E-dependent transport of the appropriate mRNAs to the cytoplasm (Shantz and Pegg, 1994; Rousseau *et al.,* 1996; Lai and Borden, 2000).

International Application No. WO 00/78803 describes eIF4E-binding peptides useful in therapy, and particularly for the induction of programmed cell death, presumably by disrupting the formation of the eIF4F complex. All these peptides comprise the amino acid sequence (motif) YxxxxLØ (wherein x is any amino acid and Ø is Leu, Met or Phe), required for binding human eIF4E (accession number NP_001959 in the GENBANK database, April 15, 2007 version).

Thus, there is an interest in the development of adequate eIF4E binding agents for use in anti-cancer therapy. There is also a need to find eIF4E binding agents possessing a distinct mechanism of action from that described above, to overcome drug resistances for instance.

Potato virus Y (PVY) is the type member of the *Potyvirus* genus, belonging to the *Potyviridae* family, the largest and economically most important group of plant viruses. Potyviruses have a so-called genome-linked VPg protein (hereinafter referred to as VPg protein or VPg) covalently attached to the 5' end of their linear nucleic acid genomes (Riechmann *et al.,* 1989 and Murphy *et al.,* 1991). The potyvirus VPg protein is implicated in virus protein synthesis, long-distance movement in plant tissue and virus replication (Lellis *et al.,* 2002; Schaad *et al.,* 2002; Fellers *et al.,* 1998). Early after potyvirus infection, the genomic RNA interacts with the host cellular machinery to initiate protein synthesis. The VPg protein seems to play an important role in this early event, because removal of VPg protein with proteinase K from the genomic RNA abolishes RNA infectivity and, in addition, decreases its translation efficiency *in vitro* (Leonard *et al.,* 2000; Herbert *et al.,* 1997). The amino acid sequence of the potato virus Y genome-linked protein VPg corresponds to the amino acid residues 336 to 523 of the amino acid sequence identified under accession number CAA82642 in the GENBANK database (November 14,2006 version). It is reproduced herein as SEQ ID NO: 2.

Recent studies have reported an interaction between the VPg protein of certain potyviruses (turnip mosaic virus, lettuce mosaic virus, tobacco vein mottling virus, potato virus Y) and the plant translation initiation factor eIF4E (Wittmann *et al.,* 1997; Duprat *et al.,* 2002; Kang *et al.,* 2005; Grzela *et al.,* 2006). It has also been shown that the VPg protein encoded by certain vertebrate viruses of the *Caliciviridae* family, such as the feline calcivirus (FCV), the lordsdale virus (LDV)) or the recently discovered murine norovirus (MNV-1) interact with the cap-binding protein eIF4E (Goodfellow *et al.,* 2005; Chaudhry *et al.,* 2006).

Grzela *et al.* (2006) have found that the interaction between PVY VPg protein and eIF4E seems to have rather low species specificity since it was also observed with yeast as well as with the insect (*S*. *frigiperda*) eIF4Es. The authors have also described that the VPg/eIF4E interaction results in some inhibition of cell-free protein synthesis in insect cells, but without leading to insect cell death. However, the PVY VPg primary amino acid sequence does not exhibit the eIF4E-binding motif YxxxxLØ described above.

Now, the Inventors have observed that, in presence of potato virus Y VPg protein, the nuclear pool of eIF4E was dramatically depleted in human cells. Thus and surprisingly, the Inventors have shown that the VPg protein immobilizes human initiation factor eIF4E in the cytoplasm, which results, contrary to what was observed previously in insect cells, in the inhibition of cell proliferation, leading to cell death.

Therefore, in a first aspect, the Inventors disclose an isolated and purified protein comprising or consisting of a VPg protein or a fragment thereof of at least 15 amino acids:
(i) having the ability to bind an eukaryote initiation factor eIF4E, preferably a mammal initiation factor eIF4E and more preferably a human initiation factor eIF4E, and
(ii) said protein, fragment thereof not comprising the amino acid motif YxxxxLØ, wherein x is any amino acid, Y is tyrosine (Tyr), L is leucine (Leu) and Ø is leucine (Leu), methionine (Met) or phenylalanine (Phe), as a medicament, preferably as an anti-cancer agent, or for use in treating cancer, preferably glioma, melanoma or a colon or lung cancer, in which the cancer cells express, preferably over-express, the initiation factor eIF4E, in a subject.

The present invention relates to an isolated and purified protein comprising or consisting of the VPg protein of SEQ ID NO: 2 or a fragment of at least 15 contiguous amino acids of the protein consisting of SEQ ID NO: 2, having the ability to bind an eukaryotic initiation factor eIF4E, said protein and fragment thereof not comprising the amino acid motif YxxxxLØ, wherein x is any amino acid, Y is tyrosine (Tyr), L is leucine (Leu) and Ø is leucine (Leu), methionine (Met) or phenylalanine (Phe), for use as a medicament, as defined in the appended claims.

A "subject" refers to a mammal, preferably a human.

The characterization of the binding (or interaction) of a protein, or a fragment thereof as defined above to an eukaryotic (*e.g.*, insect), preferably a mammal and more preferably a human eIF4E can be performed by an ELISA-based binding assay as described in Grzela *et al.* (2006). Advantageously, the dissociation constant between said protein or fragment thereof and said eIF4E is between about 5 nM and about 450 nM. Dissociation constant (or affinity) measurements may be made using methods known to those skilled in the art, including using the method described in Grzela *et al.* (2006). Optionally, this characterization may be combined with the *in vitro* determination of the change in cellular localization of eIF4E (immobilization of eIF4E in the cytoplasm) in the presence of a protein or a fragment thereof as defined above, in eukaryotic, preferably mammal and more preferably human cells. By way of example, the *in vitro* determination of the change in cellular localization of eIF4E in the presence of a protein or a fragment thereof as defined above can be performed in insect cells (*e.g.,* High Five (*Trichoplusia ni*) cells), since these cells are a good predictive model for said determination in human cells. This determination can be performed as in Examples 3 or 4 below.

The terms "protein" and "peptide" may be used interchangeably herein. They are well known in the art and refer to a polymeric form of amino acids of any length. The terms also refer to a protein or peptide comprising chemically or biochemically modified amino acids, as well as a protein or peptide having modified peptide backbones, *e.g.* a protein or peptide containing one or more modifications to L-amino acid side-chains (for example D-amino acids), or to the alpha-amino acid backbone.

A VPg protein refers usually to a virus-encoded protein covalently attached to the 5' end of the linear nucleic genome of said virus (Riechmann *et al.,* 1989 and Murphy *et al.,* 1991), and also comprises:
- an isolated plant virus-encoded VPg protein,
- a recombinant VPg protein of a plant virus, or
- a synthetic VPg protein of a plant virus.

Preferably, said VPg protein consists of about 188 to 193 amino acid residues and includes the amino acid motif KGK and NMYG (SEQ ID NO: 26).

In a preferred embodiment, the VPg protein is the potato virus Y-encoded VPg protein of SEQ ID NO: 2.

The term "plant virus" refers to a virus capable of spreading through a plant and particularly of infecting at least one type of plant cells.

According to another embodiment, the fragment of a protein as defined above is a fragment of a VPg protein or a fragment of a protein analog of a VPg protein as defined here above, comprising or consisting of at least 15, and by order of increasing preference at least 20, 25, 26 or 42 contiguous amino acids residues of said VPg protein or said analog thereof, but that retains the ability to bind an eukaryote (e.g., insect) initiation factor eIF4E, preferably a mammal initiation factor eIF4E and more preferably a human initiation factor eIF4E, as described above.

By way of example, the fragment as defined above comprises or consists of at least 15, and by order of increasing preference at least 20, 25, 26 or 42 contiguous amino acids residues of the amino acid sequence of any of the VPg protein as defined here above, preferably of SEQ ID NO: 2, 4, 6, 8, 10 or 12.

In another preferred embodiment of the present invention, the fragment of PVY VPg (SEQ ID NO: 2) comprises or consists of the amino acid sequence located:
- between the arginine (Arg) at position 41 and the arginine (Arg) at position 94 (also named VPg2; SEQ ID NO: 14) of SEQ ID NO: 2, or
- between the arginine (Arg) at position 41 and the glycine (Gly) at position 82 (also named VPg 4; SEQ ID NO: 16) of SEQ ID NO: 2,
- between the arginine (Arg) at position 41 and the phenylalanine (Phe) at position 66 (also named VPg5; SEQ ID NO: 18) of SEQ ID NO: 2,
- between the arginine (Arg) at position 41 and the arginine (Arg) at position 59 (also named VPg1; SEQ ID NO: 20) of SEQ ID NO: 2, or
- between the phenylalanine (Phe) at position 60 and the arginine (Arg) at position 94 (also named VPg3; SEQ ID NO: 22) of SEQ ID NO: 2.

In another embodiment, the fragment comprises or consists of at least 15, and by order of increasing preference at least 20, 25 or 26 contiguous amino acids residues of the amino acid sequence SEQ ID NO: 16.

Optionally, the protein or fragment thereof as defined here above may comprise an amino acid sequence facilitating cellular uptake. Such amino acid sequences, known as Cell Penetrating Peptides, are well known in the art; *See* CELL PENETRATING PEPTIDES: PROCESSES AND APPLICATIONS, edited by Ulo Langel (2002); or Advanced Drug Delivery Reviews, 2005, 57:489-660). These include the Human Immunodeficency Virus type 1 (HIV-1) protein Tat or fragment thereof (Ruben *et al.,* 1989), the herpes virus tegument protein VP22 (Elliott and O'Hare, 1997), penetratin (Derossi *et al.,* 1996), protegrin 1 antimicrobial peptide SynB (Kokryakov *et al.,* 1993) and the basic fibroblast growth factor (Jans, 1994).

In a second aspect, the Inventors disclose the use of a protein or a fragment thereof as defined here above for the manufacture of a medicament for treating cancer, preferably glioma, melanoma or a lung or colon cancer, in which the initiation factor eIF4E is expressed or over-expressed, in a subject.

In a third aspect, the Inventors disclose an isolated nucleic acid sequence encoding a protein or a fragment thereof as defined above. Said nucleic acid sequence can be synthesized using standard techniques. Techniques for nucleic acid manipulation are known in the art (*See,* for example, in Sambrook J. *et al.* (2000) Molecular Cloning: A Laboratory Manual). By way of example, the nucleic acid sequence comprises or consists of the nucleic acid sequences SEQ ID NO: 1 (residues 1007 to 1570 of the nucleotide sequence identified under accession number Z29526 in the GENBANK database (November 14, 2006 version) encoding PVY VPg protein), or SEQ ID NO: 3, 5, 7, 9, 11, 13, 15, 17, 19 or 21.

The Inventors disclose a recombinant vector for the expression of a protein or a fragment thereof as defined above. The recombinant vector comprises at least one nucleic acid sequence encoding a protein or a fragment thereof as defined above, operably linked to at least one regulatory sequence.

The term "vector" is intended to mean a nucleic acid molecule capable of transporting another nucleic acid. By way of example, a vector which can be used includes, but is not limited to, a viral vector (*e.g.*, retrovirus, adenovirus, baculovirus), a plasmid, a RNA vector or a linear or circular DNA or RNA molecule which may consist of a chromosomal, non chromosomal, semi-synthetic or synthetic nucleic acid. Large numbers of suitable vectors are known to those of skill in the art and commercially available. Preferred vectors are those capable of autonomous replication (episomal vector) and/or expression of nucleic acids to which they are operably linked (expression vectors).

The term "operably linked" is intended to mean that the nucleotide sequence is linked to a regulatory sequence in a manner which allows expression of the nucleotide sequence.

The term "regulatory sequence" includes promoters, enhancers and transcriptional or translational control elements. Such regulatory sequences are also known in the art.

By way of example, expression vectors may include an origin of replication or autonomously replicating sequence (ARS) and expression control sequences, a promoter, an enhancer and necessary processing information sites, such as ribosome-binding sites, RNA splice sites, polyadenylation sites, transcriptional and translational terminator sequences, and mRNA stabilizing sequences. Such vectors may be prepared by means of standard recombinant techniques well known in the art and discussed, for example, in Sambrook *et al.,* 2000.

The Inventors disclose the use of at least one recombinant vector expressing a nucleic acid encoding a protein or a fragment thereof as defined above, for the preparation of a medicament for preventing or treating cancer.

The expression vector can also be used to transfect or transduct (*e.g.*, by electroporation) or infect cells to thereby introduce or produce a protein or a fragment thereof as defined above.

The disclosure further describes a host cell transfected to express a protein or a fragment thereof as defined above. The host cell can be transfected with the nucleic acid or the vector as defined above. The host cell may be any procaryotic or eucaryotic cell. For example, a protein or a fragment thereof as defined above may be expressed in bacterial cells such as *E. coli,* insect cells, yeast, mammalian cells such as Chinese hamster ovary cells (CHO) or animal or human cells such as B16, LGL26, HeLa or 293 or any transformed cell line.

In another aspect, the Inventors disclose a pharmaceutical composition comprising a protein or a fragment thereof or a recombinant vector as defined here above and a pharmaceutically acceptable carrier. Said composition is useful for treating cancer, preferably glioma, melanoma or a colon or lung cancer, in which the initiation factor eIF4E is expressed or over-expressed, in a subject.

As used herein, "pharmaceutically acceptable carrier" is intended to include any and all solvents, dispersion media, liposomes, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field. Preferred examples of such carriers or diluents include, but are not limited to, water, saline, Ringer's solutions, dextrose solution, and 5% human serum albumin. Cationic lipids, non-aqueous vehicles such as fixed oils and commercially available transductants may also be used. The use of such media and agents for pharmaceutically active substances is well known in the art. Except insofar as any conventional media or agent is incompatible with a protein, a fragment thereof or an analog thereof as defined hereabove, use thereof in the composition as defined above is contemplated.

By way of example, when the pharmaceutical composition comprises a protein or a fragment thereof, then the pharmaceutically acceptable carrier is preferably a cationic lipid, or a mixture of non-cationic lipids and a cationic lipid.

In yet another aspect, the description discloses a method of inhibiting cancer cell proliferation, characterized in that said method comprises the step of treating a subject in need thereof with a composition comprising a protein or a fragment thereof as defined here above.

The term "inhibiting" refers to slowing, decreasing, delaying, preventing or abolishing cell proliferation.

The term "cell proliferation" refers to the rate at which a group of cells divides. The number of cells growing can be easily quantified by one skilled in the art.

The term "cancer" refers to any of a number of diseases that are characterized by uncontrolled, abnormal proliferation of cells, the ability of affected cells to spread locally or through the bloodstream and lymphatic system to other parts of the body (*i.e.,* metastases), as well as any of a number of characteristic structural and/or molecular features. Examples of cancers are breast, colorectal, liver, lung (such as small cells, non-small cells), bronchic, prostate, ovarian, brain, pancreatic, colon, head and neck, stomach and bladder cancers, non-Hodgkin's lymphomas, melanomas, leukaemias, neuroblastomas, gliomas, or glioblastomas.

A "cancer cell" is understood as a cell having specific structural properties, which can lack differentiation and be capable of invasion and metastasis. Preferably, the cancer cells express or over-express the initiation factor eIF4E. Cancer cells expressing eIF4E can be identified by methods known in the art, *e.g.,* by DNA sequencing, or by Northern blot, or by immunochemistry using anti-eIF4E antibodies.

The term "over-express" means that the gene encoding eIF4E protein is expressed at a higher level compared to the one of a normal (*i.e.,* non cancer cell) quiescent eukaryotic, mammal or human cell, resulting in the production in a cancer cell with eIF4E level exceeding the one normally produced in said normal cell. The normal range of expression or production can be determined by routine methods as by assaying the eIF4E protein, its mRNA, or its gene.

The term "treating" includes the administration of the protein or a fragment thereof as defined here above, or a composition as defined above, to a patient who has a disease or disorder (*e.g.*, cancer or metastatic cancer), a symptom of disease or disorder or a predisposition toward a disease or disorder, with the purpose to cure, heal, alleviate, relieve, alter, remedy, ameliorate, improve or affect the disease or disorder, the symptoms of the disease or disorder, or the predisposition toward disease, or extend duration of life.

In another aspect, the Inventors disclose the use of an isolated and purified initiation factor eIF4E, preferably from human origin, to screen for compounds (*e.g.*, drugs) that mimic the binding specificity of a VPg protein, preferably the VPg protein of SEQ ID NO: 2, with eIF4E.

In another aspect the Inventors disclose a method of screening for compounds (*e.g.*, drugs) which mimic the binding specificity of a VPg protein, preferably of SEQ ID NO: 2, 4, 6, 8, 10 or 12, and more preferably of SEQ ID NO: 2, with the initiation factor eIF4E (preferably the human eIF4E), comprising the steps of i) contacting an isolated and purified eIF4E with a candidate compound and a protein, a fragment thereof or an analog thereof as defined above (*e.g.*, SEQ ID NO: 2) and ii) detecting if this candidate compound inhibits the binding (or interaction) between eIF4E and said protein or a fragment thereof Standard methods are available in the art to carry out competitive binding assays. In addition, the detection of the binding (or interaction) of a peptide compound with eIF4E can be carried out as described in Grzela *et al.* (2006).

Candidate compounds include peptides such as soluble peptides, lipids, carbohydrates, lipopeptides, glycopeptides, and small organic and inorganic molecules.

The Table 1 below sums up the nucleic acid and peptide sequences used in the present description.

| SEQ ID NO: | Description of the sequence |
|---|---|
| 1 | nucleic acid sequence encoding PVY VPg protein |
| 2 | PVY VPg protein |
| 3 | nucleic acid sequence encoding TEV VPg protein |
| 4 | TEV VPg protein |
| 5 | nucleic acid sequence encoding CIYVV VPg protein |
| 6 | ClYVV VPg protein |
| 7 | nucleic acid sequence encoding TVMV VPg protein |
| 8 | TVMV VPg protein |
| 9 | nucleic acid sequence encoding TuMV VPg protein |
| 10 | TuMV VPg protein |
| 11 | nucleic acid sequence encoding LMV VPg protein |
| 12 | LMV VPg protein |
| 13 | nucleic acid sequence encoding VPg2 |
| 14 | VPg2: amino acid sequence located between the arginine (Arg) at position 41 and the arginine (Arg) at position 94 of SEQ ID NO: 2 |
| 15 | nucleic acid sequence encoding VPg4 |
| 16 | VPg4: amino acid sequence located between the arginine (Arg) at position 41 and the glycine (Gly) at position 82 of SEQ ID NO: 2 |
| 17 | nucleic acid sequence encoding VPg5 |
| 18 | VPg5: amino acid sequence located between the arginine (Arg) at position 41 and the phenylalanine (Phe) at position 66 of SEQ ID NO: 2 |
| 19 | nucleic acid sequence encoding VPg1 |
| 20 | VPg1: amino acid sequence located between the arginine (Arg) at position 41 and the arginine (Arg) at position 59 of SEQ ID NO: 2 |
| 21 | nucleic acid sequence encoding VPg3 |
| 22 | VPg3: amino acid sequence located between the phenylalanine (Phe) at position 60 and the arginine (Arg) at position 94 of SEQ ID NO: 2 |
| 23 | PVY VPg gene forward primer |
| 24 | PVY VPg gene reverse primer |
| 25 | polyhedrin promotor of AcMNPV |
| 26 | VPg motif NMYG |
| 27 | FCV VPg protein |
| 28 | HuNoV VPg protein |
| 29 | HuNoV VPg protein |
| 30 | MNV VPg protein |

In addition to the preceding features, the invention further comprises other features which will emerge from the following description, which refers to examples illustrating the present invention, as well as to the appended figures.

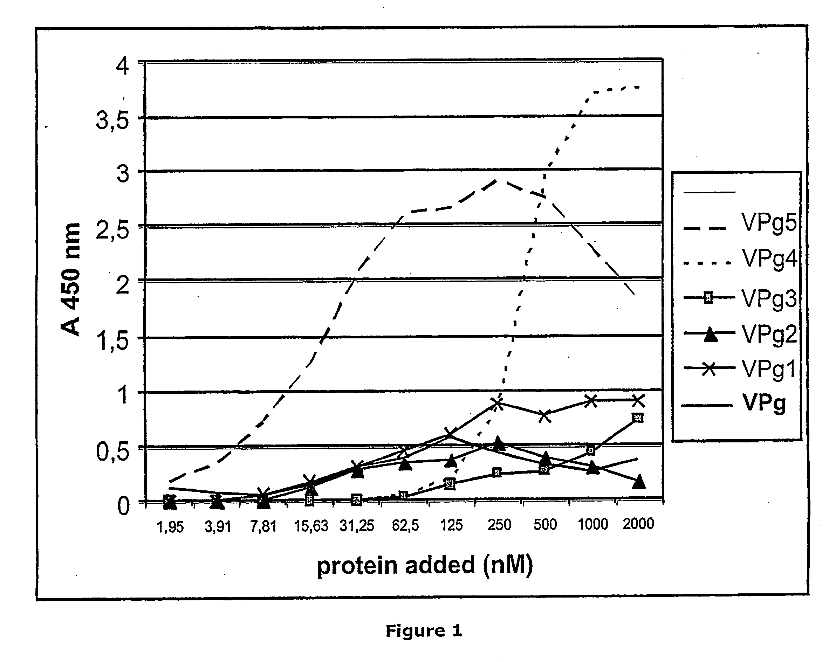

**Figure 1****:** Interaction assay between human eIF4E and PVY VPg protein or fragments thereof (VPg1 to VPg4).

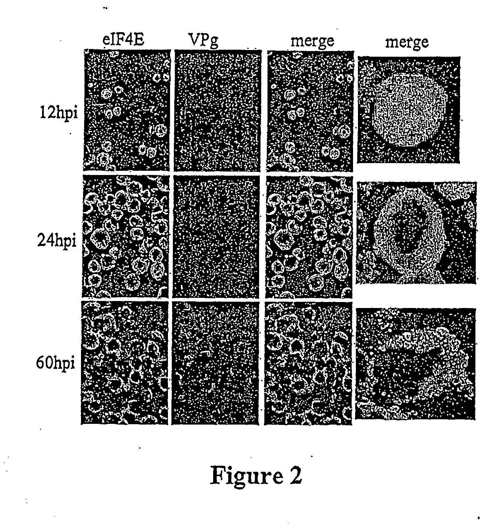

**Figure 2****:** Effect of VPg protein (SEQ ID NO: 2) expression on localization of native elF4E in insect cells. VPg was expressed in insect cells and distribution of native elF4E (left column) and recombinant VPg (VP column) was analyzed by confocal microscopy at the indicated timepoints post infection. The blow-ups of some cells are shown in the right column.

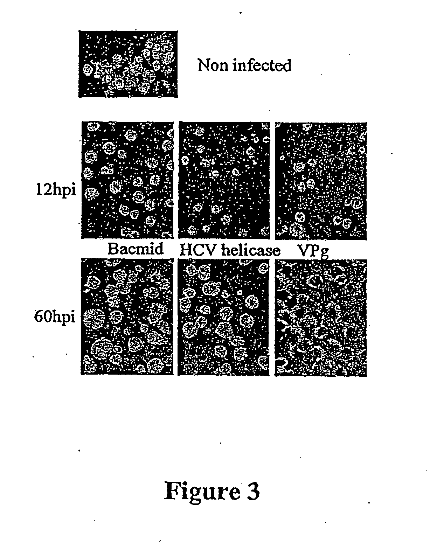

**Figure 3****:** Cytoplasmic retention of native elF4E is specific to VPg protein (SEQ ID NO: 2). Insect cells have been infected with bacmid (null baculovirus, left column), domain II of HCV helicase (middle column) and VPg (right column). Insect cells were analyzed by confocal microscopy at the indicated timepoints post infection.

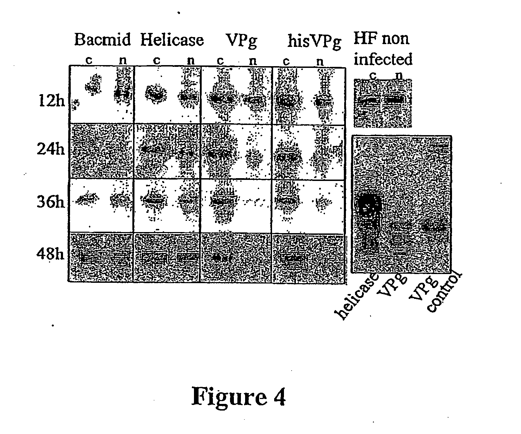

**Figure 4****:** elF4E expression in fractionated insect cells. Insect cells infected at MOI 10 with appropriate baculoviruses were collected at indicated timepoints post infection, nuclei were separated from the cytoplasm and the subcellular extracts were analysed by SDS/PAGE and Western blot. The expression of the recombinant proteins is shown on the far right lower panel.

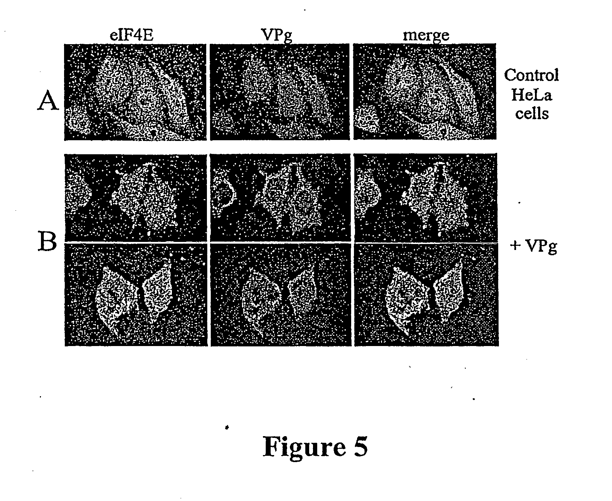

**Figure 5****:** Effect of PVY VPg protein on human eIF4E localisation. HeLa cells were transduced with VPg in the presence of Pro-Ject^{Tm} (purchased from Pierce) and analysed for presence of VPg and eIF4E by confocal miscroscopy (Figure **1B**). In Figure **1A** the HeLa cells were not transduced.

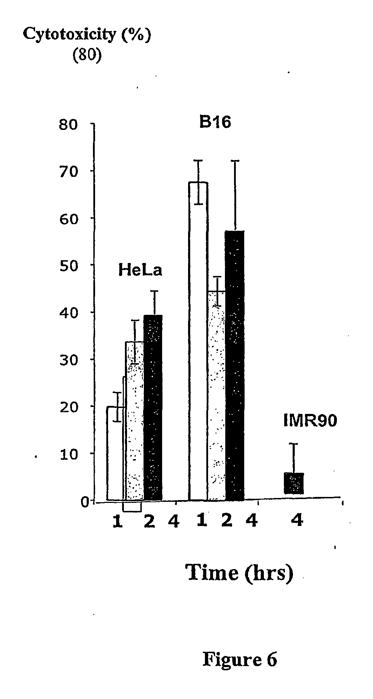

**Figure 6****:** Effect of PVY VPg protein on human cell proliferation. Cells were transduced with VPg using as protein transfer reagent TransPass P (Ozyme), and incubated for indicated periods. Cells damage was estimated using LDH Cytotoxicity detection assay (Clontech). The results show the average result obtained from three wells.

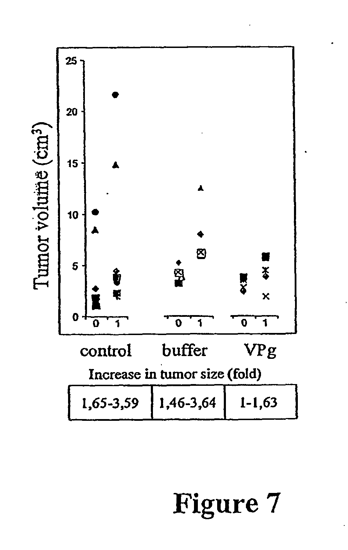

**Figure 7****:** Antitumour efficacy of PVY VPg protein in mice carrying GL26 (mouse glioma) tumours. 0 = tumour volume measured at the beginning of the experiments. no treatment, 1 = tumour volume measured 3 days later. Control = untreated mice. Buffer = mice electroporated with 0.9% NaCl. VPg = mice electroporated with 25 µg VPg in 0.9% NaCl. The same sign at the day 0 and 3 days later shows the volume of the tumour in the same mouse. The figure also give the fold increase in tumour size between time 0 and time 1 (fold 1 means no increase in tumour size).

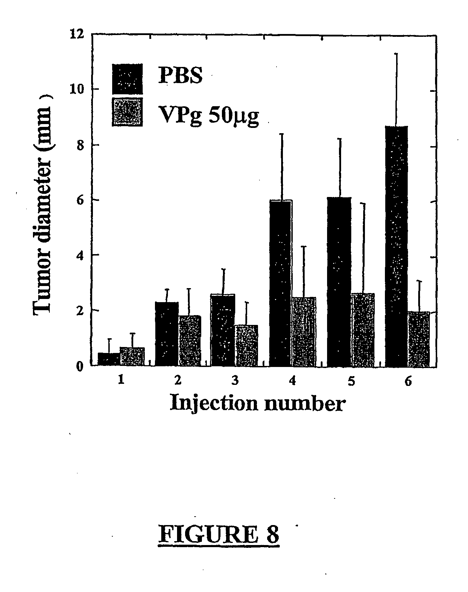

**Figure 8****:** Antitumour efficacy of PVY VPg protein in mice carrying B16-ova (murine melanoma) tumours. B16-ova melanoma cells were injected into the thigh of the C57BL/6 mice (two groups of 8 animals). Two weeks later palpable tumors were formed. Control group was injected into the tumor with buffer mixed with Transpass P. Second group had injections of 50 micrograms of VPg transduced with Transpass P. every day for 6 days. The diameter of the tumours were measured every day during six days of treatment.

### EXAMPLES

### Example 1: Materials and Methods

### Cells.

High-Five insect (*Trichoplusia ni*) cells grown in suspension were cultured in Express Five SFM medium (Invitrogen) with gentamicin (50 mg/ml) and amphotericin B (0.25 mg/ml).

HeLa (human cervix carcinoma), B16-ova (mouse melanoma), GL26 (mouse glioma), IMR90 (human primary diploid fibroblasts) were grown in DMEM+GlutaMAX^{™}-I (Gibco) medium complemented with 10% FCS.

**Antibodies.** Anti-his mouse monoclonal antibody (MMS-156P) was purchased from BabCO (Berkeley, CA, USA). Anti-eIF4E mouse monoclonal (P-2; sc-9976) was from Santa Cruz Biotechnology (Santa Cruz, CA, USA). It recognizes mouse, rat, human and porcine eIF4E. Anti-eIF4E rabbit polyclonal antibody against human eIF4E (purchased from Jackson ImmunoResearch Laboratories) was kind gift of Nahum Sonenberg (McGill University, Montreal Canada).

Anti-VPg rabbit polyclonal antibody was prepared in Elevage Scientifique des Dombes (Chatillon, France), using as antigen PVY hisVPg expressed in baculovirus (described below) and was affinity purified to VPg as follows. First an acetone powder obtained from 1 g of non-infected insect cells was used to remove cross-reacting antibodies from serum. Cell powder was added to the anti-VPg rabbit serum up to final concentration of 1%, and stored on ice overnight with gentle rolling. After centrifugation at 10 000 g for 10 min at 4° C, supernatant fraction was applied to CNBr-activated Sepharose 4B (purchased from Amersham Bioscience) containing VPg protein immobilized according to the manufacturers instructions. Bound antibody was eluted with 100 mM glycine pH 2.4 directly into tubes containing 30 µl of 3 M Tris, pH 8.8 and 20 µl 5 M NaCl.

**Recombinant protein expression and purification.** VPg gene of potato virus Y (strain O, accession number Z29526 in GENBANK database) was synthesized by PCR using plasmid pPVY15 (available under accession number Z29526 in GENBANK database, November 14, 2006 version) as template, with the forward primer 5' GGGGGGGATCCATGGGGAAAATAAA-3' (SEQ ID NO: 23) and the reverse primer 5' CCCCCAGATCTCTATTATTCATGCTCC-3' (SEQ ID NO: 24, permitting the expression of the protein with the accession number CAA82642 in the GENBANK database. VPg cDNA was inserted into pFastBac HTb (purchased from Invitrogen, Carlsbad, CA, USA) under the control of polyhedrin promotor (SEQ ID NO: 25) of AcMNPV (O'Reilly *et al.,* 1992) and the recombinant baculovirus expressing his-tagged VPg was constructed using bacmid technology (purchased from Gibco BRL). The High Five (*Trichoplusia ni*) cells in suspension were infected with the recombinant baculovirus at MOI of 5 pfu/cells. Portion (100 ml) of recombinant baculovirus-infected HF cells was collected at 72h p.i. suspended in 10 ml of 50 mM phosphate buffer, pH 7.0, containing 300 mM NaCl., 6 mM β-mercaptoethanol, 5% glycerol, 0.5% Tween 20 and protease inhibitor cocktail (Complete, purchased from Roche) and lysed by five cycles of freezing in liquid nitrogen and thawing at 37°C. After clearing the extract was passed by gravity through 1 ml of Ni-NTA agarose resin (purchased from Qiagen), equilibrated with 5 ml of lysis buffer. Bound proteins were eluted 5 x 500 µl of 250 mM imidazole in the 50 mM phosphate buffer, pH 7.0, containing 300 mM NaCl. Fusion tag was removed from VPg with TEV protease for 48 h incubation at 10°C. The resulting products were purified on 5 ml Hi-Trap Heparin column (purchased from Amersham Pharmacia). Proteins were applied on the column in 100 mM NaCl in 50 mM phosphate, pH 7.0, washed with the same buffer until the OD₂₈₀ reached stable level and then eluted in the gradient of NaCl in 50 mM phosphate, pH 7.0 (from 100 mM to 1 M). The fractions containing full-length hisVPg were collected, diluted and fractionated on 5 ml 15 S-Source column (purchased from Amersham Pharmacia) under conditions used for Hi-Trap Heparin column. His-tagged domain II of HCV helicase was expressed in HF cells after infection with the appropriate baculovirus at MOI 5 and purified as described (Boguszewska-Chachulska *et al.,* 2004). All steps were analyzed on 12% SDS-PAGE by staining with CBB, using Precision Plus Protein Dual Colour (purchased from Bio-Rad) and BenchMark Prestained Protein Ladder (purchased from Invitrogen) as molecular weight standards.

**Cell fractionation.** High Five (*T. ni*) cells at the concentration 2x10⁶ cells/ml were infected with the baculoviruses expressing VPg protein or hisVPg, or domain II of HCV helicase or empty bacmid, at MOI 5. The infected cells were collected at the indicated times p.i. by centrifugations at 2000 rpm for 10 min. The pellets were washed twice in PBS and resuspended in four times the packed cell volume of hypotonic buffer (10 mM Tris, pH 7.9, 10 mM KCl, 3 mM DTT, 0.1 mM EDTA, 0.1 mM EGTA, 0.75 mM spermidine, 15 mM spermine). The cells were allowed to swell on ice for 30 min and checked with phase microscope for complete breakage. Then a 1/10 volume of restoration buffer (50 mM Tris, pH 7.9, 0.75 mM spermidine, 0.15 mM spermine, 10 mM KCl, 0.2 mM EDTA, 3 mM DTT, 67.7% sucrose) was added and the homogenate was layered over a 1 ml of sucrose cushion (30% sucrose in hypotonic buffer) and centrifuged in cold for 20 min at 3000 rpm. The pelleted nuclei were resuspended in four times the packed cell volume of nuclear extraction buffer (50 mM Tris, pH 7.5, 0.42 mM KCl, 6 mM DTT, 0.1 mM EDTA, 10% sucrose, 5 mM MgCI₂ 20% glycerol, 0.5 mM PMSF, 3 mg leupeptin per ml). The nuclei were then lysed by gentle rocking at 4°C for 30 min at 4°C. Cytoplasmic and nuclear fractions were run on 15% SDS-PAGE followed by Western blot with anti-eIF4E antibody using ECL system with X-ray film. Densitometry of eIF4E bands was performed using GelDoc 2000 (purchased from BioRad) with Quantity One software.

**Cell transduction with VPg protein.** Transpass P (Ozyme) was combined with the purified VPg (0.1-2 µg/well) in 20 mM sodium phosphate, pH 7.0, containing 150 mM NaCl or in PBS according to the manufacturer instructions. Serum-free medium was added to the VPg/Transpass P mixture up to 250 µl final volume. HeLa cells (10⁵/well of 24-well dish) prewashed with serum-free medium were treated with such portion of delivery mixture. After indicated periods of incubation at 37°C cells were used for studies on protein localization (confocal microscopy) and on proliferation (LDH Kit from Clontech).

**Localization of elE4E and VPg in insect cells by confocal microscopy.** High Five (*T. ni*) cells in suspension (2x10⁵ cells/ml) were infected with 10 MOI of baculoviruses expressing either hisVPg, or his-tagged domain II of HCV helicase or empty bacmid. Cells were collected at 48 h p.i by centrifugation at 3000 rpm, 5 min, 4°C, followed by two washes with 500 µl of PBS. Cells were fixed in cold 2% PFA, left for 10 min at RT, washed two times with 500 µl of PBS and resuspended in 300 µl of PBS. Portions of 100 µl of fixed cells were applied onto clean round coverslips and allowed to attach while drying in a lamina flow hood. Next day cover slips were put into wells of 24-wells plate and rehydrated at room temperature with 500 µl portions of PBS. After PBS removal, cells were permeabilized for 10 min with cold 0.1% Triton X-100 in PBS, rinsed twice with PBS and blocked with 5% serum in PBS for 30 min at RT. Alter 2 washes with PBS cells were incubated for 1 h at RT with primary antibodies (anti-his or anti-human elF4E) diluted 1/100 in PBS, followed by washing and 1h incubation with secondary antibody (anti-rabbit FITC or anti-mouse Texas Red) (Jackson ImmunoResearch Laboratories) diluted 1:100 in PBS. Nuclei were stained with propidium iodide diluted in PBS (0.2-1 µg/ml). Alter three PBS rinses cover slips were mounted with 50% glycerol. Images were collected with BioRad MRC-600/Nikon Optiphot laser scanning confocal microscope.

**Localization of eIF4E, VPg protein and PML in human cells by confocal microscopy.** HeLa cells seeded on cover slips at about 60 % confluency were washed 3 times with PBS and fixed in 2% cold PFA. Cells were infected with 10 MOI of baculoviruses expressing either hisVPg, or his-tagged domain 2 of HCV helicase or empty bacmid. Cells were collected at 48 h p.i by centrifugation at 3000 rpm, 5 min, 4°C, followed by two washes with 500 µl of PBS. Cells were fixed in cold 2% PFA, left for 10 min at Room Temperature, washed two times with 500 µl of PBS and resuspended in 300 µl of PBS. Portions of 100 µl of fixed cells were applied onto clean round coverslips and allowed to attach while drying in a lamina flow hood. Next day cover slips were put into wells of 24-wells plate and rehydrated min at room temperature with 500 µl portions of PBS. After PBS removal, cells were permeabilized for 10 min with cold 0.1% Triton X-100 in PBS, rinsed twice with PBS and blocked with 5% serum in PBS for 30 min at RT. After 2 washes with PBS cells were incubated for 1 h at room-temperature with primary antibodies (purified polyclonal anti-VPg, anti-eIF4E (MAb) and anti-PML (MAb) antibodies) diluted 1/100 in PBS, followed by washing and 1 h incubation with secondary antibody (anti-rabbit FITC or anti-mouse Texas Red) (purchased from Jackson ImmunoResearch Laboratories) diluted 1:100 in PBS. Nuclei were stained with propidium iodide diluted in PBS (0.2-1 µg/ml). After three PBS rinses cover slips were mounted with 50% glycerol. Images were collected with BioRad MRC-600/Nikon Optiphot laser scanning confocal microscope.

### Effect of VPg protein on cell proliferation.

HeLa, B16, GL26 and IMR90 cells were seeded in 96-wells plate at 1x10⁴ cells per well and cultivated overnight. Portion of VPg (0.5-2 µg/well) was diluted into serum-free medium to obtain total volume 10µl and incubated for 20 min with 0.2µl TransPass P Protein Transfection Reagent (New England BioLabs) at room temperature. Cells were rinsed with serum-free medium and covered with 100µl/well of mixture containing serum-free medium and transduction mix. After various time of incubation at 37°C time the plates were centrifuged at 1000 rpm for 10 min and the supernatants were transferred to the new plate. The release of the malate dehydrogenase was measured with the LDH Cytotoxicity Detection Kit (Clontech), using multilabeled counter Victor1412 (Wallac) at 490nm. The level of cytotoxicity was calculated according to the LDH kit manual.

### Example 2: In vitro interaction assay of human eIF4E with PVY VPg protein and fragments thereof

Peptides SEQ ID NO: 2 (VPg), SEQ ID NO: 14 (VPg2), SEQ ID NO: 16 (VPg4), SEQ ID NO: 20 (VPg1), SEQ ID NO: 22 (VPg3) and SEQ ID NO: 18 (VPg5) were obtained by expression in *E. coli* as GST-fusion proteins. GST-fusion proteins were produced by standard methods from cDNA sequences (encoding VPg, VPg1, VPg2, VPg3, VPg4 or VPg5) cloned into vector pGEX-4T-1 (purchased from Amersham Biosciences).

ELISA technique was used for measuring the peptide's interaction with eIF4E immobilised in wells of 96-well dish. The interaction was monitored with an anti-GST antibody.

Human eIF4E, 100 ng in the coating buffer (0.1 M carbonate buffer, pH 9.6) was applied to 96-wells micro titer plate for overnight at 4°C. Excess eIF4E was removed and the wells were blocked with 5% milk in the PBST buffer for 1 h at 37°C. The plate was rinsed three times with PBST and incubated with increasing quantity of the various GST-fusions peptides in PBST, for 1 h at RT. After two PBST washes the incubation with anti-GST HRP- conjugated antibody (1:5000) (purchased from Amersham Biosciences) was carried out for 30 min at 37°C. After three PBST washes the reaction was developed with peroxides substrate and the absorbance was measured at 490 nm. Non-specific interactions of GST protein and anti-GST antibody with the GST-fusion peptides were substracted.

Results are shown in Figure 1. Among the peptides tested, it appeared that SEQ ID NO: 22 (VPg3) is the weaker interactor. SEQ ID NO: 20 (VPg1) and SEQ ID NO: 14 (VPg2) showed approximately similar level of interaction, which is not far from VPg itself. The best eIF4E-interacting peptide seems to be SEQ ID NO: 16 (VPg4) and SEQ ID NO: 18 (VPg5), which, at higher concentration functions better than PVY VPg protein itself (SEQ ID NO: 2).

### Example 3: In vitro cellular localisation of elF4E and PVY VPg protein (SEQ ID NO: 2) in insect cells

Insect cells have been infected with baculovirus containing VPg gene of PVY (SEQ ID NO: 1). Confocal microscopy analysis revealed that the initiation factor elF4E which localized at the beginning of infection in both compartiments, the nucleus and the cytoplasm (Figure 2, 12hpi), at 60 hpi timepoint was observed at the cell periphery (Figure 2, left columns). VPg was observed only in the cytoplasm throughthout the infection (Figure 2, VPg column). Merged images showed significant fraction of the elF4E in the nucleus at the beginning of infection but which diminished in the course of infection; at 24hpi, the elF4E was visible in the cytoplasm and just at the nucleus periphery and at 60hpi was observed in the cytoplasm only, showing significant overlap with the VPg.

To find out if the observed fluctuations in the elF4E localization are specifically due to the presence of VPg, it was investigated the localization of elF4E upon insect cells infection by baculovirus null (bacmid) which is a virus of origin, before insertion of the foreign gene, and also upon infection with baculovirus expressing non-relevant protein, the domain II of HCV helicase or VPg (Figure 3). In non-infected insect cells elF4E was observed in both cellular compartments (the most upper panel). At 12 hpi cells infected with all three kinds of virus behaved similarly, whereas al 60hpi only cells expressing VPg showed elF4E retention in the cytoplasm (Figure 3, lowest panel). To obtain more quantitative data, the cell nucleus was separated from the cytoplasm, and elF4E quantity in both compartiments was assessed by Western blot (Figure 4) followed by densitometry (Table 2 below).

**Table 2: eIF4E distribution in insect cell cytoplasm and nucleus**

| Hours p.i. | baculovirus | eIF4E in cytoplasm (%) | eIF4E in nucleus (%) |
|---|---|---|---|
| HF cells | none | 48.49 | 51.51 |
| 12h | bacmid | 46.5 | 53.5 |
| | HCV helicase domain | 51.54 | 48.46 |
| | VPg | 55.5 | 44.5 |
| | hisVPg | 53.45 | 46.55 |
| 24h | bacmid | 57.83 | 42.17 |
| | HCV helicase domain | 63.05 | 36.95 |
| | VPg | 84.74 | 15.26 |
| | hisVPg | 81.3 | 18.7 |
| 36h | bacmid | 59.2 | 40.8 |
| | HCV helicase domain | 62.5 | 37.5 |
| | VPg | 87.1 | 12.9 |
| | hisVPg | 83.33 | 16.67 |
| 48h | bacmid | 55.99 | 44.01 |
| | HCV helicase domain | 57.9 | 42.1 |
| | VPg | 73.9 | 26.1 |
| | hisVPg | 74.07 | 25.93 |

Cells infected with the baculovirus expressing his-tagged form of VPg were included in the experiment. In both non infected as well as cells infected for 12h, comparable level of elF4E were present in the cytoplasm and the nucleus. The nuclear pool of the initiation factor diminished somewhat in the course of infection with bacmid and with helicase-expressing virus. However, upon expression of both VPg forms the nuclear pool of the elF4E was severely depleted; at 36hpi the nuclear pool of elF4E accounted for 13% only of total cellular factor (down from 51-53%).

These experiments demonstrated that (1) VPg expressed alone in the baculovirus system remains in the cytoplasm; (2) in the presence of VPg, elF4E is retained in the cytoplasm, resulting in the depletion of the initiation factor nuclear pool, but no inhibition of cell proliferation or cell death was observed probably because the insects cells contains several eIF4E isoforms.

### Example 4: In vitro cellular localization of eIF4E and PVY VPg protein in HeLa cells

In order to know if VPg protein of plant virus is able to interact with human eIF4E, and resulting in the depletion of the nuclear pool of the initiation factor, several tranfection experiments were undertaken under various conditions. However, no cells expressing VPg could be recovered, suggesting toxic effect of VPg on human cells. Therefore the PVY VPg (SEQ ID NO: 2) was directly translocated into HeLa cell with Pro-Ject^{Tm} (purchased from Pierce), a cationic lipid mixture which allows intracellular delivery of proteins (see Figure 5B). VPg was localized in the cytoplasm and affected strongly localization of native eIF4E. The initiation factor in control HeLa cells was observed in cytoplasm and nucleus. In the presence of VPg the level of eIF4E diminished significantly in the nucleus and overlapped to large extent with the cytoplasm-localized VPg.

### Example 5: Effect of PVY VPg protein on cell proliferation in vitro

The LDH assay, which permits evaluation of cell damage was applied after VPg delivery to transformed cells, HeLa and B16 and to primary non-transformed human cells IMR90. Already 1 hour after VPg intracellular delivery both HeLa and B16 showed significant cell damage, Importantly, VPg introduced into primary IMR90 (human fetal lung fibroblasts) did not affect their growth (Fig. 6, IMR90).

The interaction of VPg with the eukaryotic initiation factor eIF4E results in the immobilization of the initation factor in the cytoplasm which leads to inhibition of cell growth followed by cell death. It is conceivable that the decrease of the eIF4E nuclear pool caused by interaction with VPg is accompanied by inhibition of the cytoplasmic transport of the messenger RNA of the pro-proliferative proteins, leading to the inhibition of cell growth with ensuing cell death.

### Example 6: Efficacy of PVY VPg protein on cell proliferation in vivo

To study anticancer effect of PVY VPg protein (SEQ ID NO: 2), *in* vivo experiences have been undertaken. C57BL/6 mice were inoculated with GL26 cells inducing the mouse glioma tumours or with B16-ova melanoma cells.
- GL26 tumours were established by injecting portions of 0.5 x 10⁶ GL26 cells in 100 µl foetal bovine serum into the right quadriceps of the adult female C57BL/6 mice. About two weeks later, during the exponential phase of glioma tumour growth, the VPg (25 µg in 0.9% NaCl) or 0.9% NaCl (50µl) (buffer) were administered by electroporation to 2 groups of 5 mice, leaving the control group of mice untreated. VPg and buffer treated mice were anaesthetized by intraperitoneal injection of 250 µl of a solution containing 400 µl of Imalgen 1000, 2% Rompun (both from Centravet, Lapalisse, France) and 0.9% NaCl. Electroporation conditions were the following: 5 x 100 µsec pulses of the 800 volts from electro square porator T820 (BTX, San Diego, CA, U.S.A), at 1 Hz frequency. The delay between pulses was one second. The animals were kept warm (37°C) until they recovered from anaesthesia. The volume of the tumours was measured in mm³ every 2 or 3 days by slide calliper and compared with normally growing glioma tumors of the control untreated group of mice.
- B16-ova melanoma tumours were established by injection of 5 x10⁵ cells suspended in PBS into the left thighs of the C57BL/6 mice (two groups of 8 animals). After 12 days, when palpable tumors were formed, the tumours of the control group were injected with Transpass P mixed with the buffer. Second group had the injections of 50 micrograms of VPg transduced with Transpass P, every day for 6 days. The kinetics of tumour growth was followed by measuring every day the tumor diameter.

Results are shown in Figures 7 and 8. Administration of VPg slowed down or inhibited the growth of the tumours in comparison with control groups. This shows that VPg has indeed an inhibitory effect on tumour proliferation.

### REFERENCES

- Boguszewska-Chachulska AM, Krawczyk M, Stankiewicz A, Gozdek A, Haenni AL, Strokovskaya L. (2004) Direct fluorometric measurement of hepatitis C virus helicase activity. FEBS Lett. 567:253-6.
- Chaudhry Y, Nayak A, Bordeleau ME, Tanaka J, Pelletier J, Belsham GJ, Roberts LO, Goodfellow IG. (2006) Caliciviruses differ in their functional requirements for eIF4F components. J. Biol Chem. 281:25315-25.
- Derossi D, Calvet S, Trembleau A, Brunissen A, Chassaing G, Prochiantz A. (1996) Cell internalization of the third helix of the Antennapedia homeodomain is receptor-independent. J. Biol Chem. 271:18188-93.
- Duprat A, Caranta C, Revers F, Menand B, Browning KS, Robaglia C. (2002) The Arabidopsis eukaryotic initiation factor (iso)4E is dispensable for plant growth but required for susceptibility to potyviruses. Plant J. 32:927-34.
- Elliott G and O'Hare P. (1997) Intercellular trafficking and protein delivery by a herpesvirus structural protein. Cell 88:223-33.
- Fellers, J, Wan J, Hong Y, Collins GB, and Hunt AG. (1998). In vitro interactions between a potyvirus-encoded, genome-linked protein and RNA-dependent RNA polymerase. J. Gen. Virol. 79:2043-2049.
- Goodfellow I, Chaudhry Y, Gioldasi I, Gerondopoulos A, Natoni A, Labrie L, Laliberte JF, Roberts L. (2005) Calicivirus translation initiation requires an interaction between VPg and eIF4E. EMBO Rep. 6: 968-972.
- Grzela R, Strokovska L, Andrieu JP, Dublet B, Zagorski W, Chroboczek J. (2006) Potyvirus terminal protein VPg, effector of host eukaryotic initiation factor eIF4E Biochimie 88:887-96.
- Herbert TP, Brierley I, Brown TD. (1997) Identification of a protein linked to the genomic and subgenomic mRNAs of feline calicivirus and its role in translation. J. Gen Virol. 78 :1033-40.
- Jans DA. (1994) Nuclear signaling pathways for peptide ligands and their membrane receptors? Faseb J. 8 :841-847
- Kang BC, Yearn I, Frantz JD, Murphy JF, Jahn MM. (2005) The pvr1 locus in Capsicum encodes a translation initiation factor eIF4E that interacts with Tobacco etch virus VPg. Plant J. 42:392-405.
- Kokryakov VN, Harwig SS, Panyutich EA, Shevchenko AA, Aleshina GM, Shamova OV, Korneva HA, Lehrer RI. (1993) Protegrins: leukocyte antimicrobial peptides that combine features of corticostatic defensins and tachyplesins. FEBS Bett. 327:231-6.
- Lai HK, Borden KL, (2000) The promyelocytic leukemia (PML) protein suppresses cyclin D1 protein production by altering the nuclear cytoplasmic distribution of cyclin D1 mRNA. Oncogene 19:1623-34.
- Lejbkowicz F, Goyer C, Darveau A, Neron S, Lemieux R, Sonenberg N. (1992) A fraction of the mRNA 5' cap-binding protein, eukaryotic initiation factor 4E, localizes to the nucleus. Proc Natl Acad Sci U S A. 89:9612-6.
- Lellis AD, Kasschau KD, Whitham SA, Carrington JC. (2002) Loss-of-susceptibility mutants of Arabidopsis thaliana reveal an essential role for eIF(iso)4E during potyvirus infection. Curr Biol. 12:1046-51
- Leonard S, Plante D, Wittmann S, Daigneault N, Fortin MG, Laliberte JF. (2000) Complex formation between potyvirus VPg and translation eukaryotic initiation factor 4E correlates with virus infectivity. J Virol. 74:7730-7.
- Murphy JF, Rychlik W, Rhoads RE, Hunt AG, Shaw JG. (1991) A tyrosine residue in the small nuclear inclusion protein of tobacco vein mottling virus links the VPg to the viral RNA. J Virol. 65:511-3.
- O'Reilly DR, Miller LK and Luckow VA. (1992) Baculovirus Expression Vectors: A Laboratory Manual. (New York, N.Y.: W.H. Freemean and Company).
- Riechmann JL, Lain S, Garcia JA. (1989) The genome-linked protein and 5' end RNA sequence of plum pox potyvirus. J Gen Virol. 70:2785-9.
- Rosenwald IB, Kaspar R, Rousseau D, Gehrke L, Leboulch P, Chen JJ, Schmidt EV, Sonenberg N, London IM. (1995) Eukaryotic translation initiation factor 4E regulates expression of cyclin D1 at transcriptional and post-transcriptional levels. J Biol Chem. 270:21176-80.
- Rousseau D, Kaspar R, Rosenwald I, Gehrke L, Sonenberg N. (1996) Translation initiation of ornithine decarboxylase and nucleocytoplasmic transport of cyclin D1 mRNA are increased in cells overexpressing eukaryotic initiation factor 4E. Proc Natl Acad Sci U S A. 93:1065-70.
- Ruben S, Perkins A, Purcell R, Joung K, Sia R, Burghoff R, Haseltine WA, Rosen CA. (1989) Structural and functional characterization of human immunodeficiency virus tat protein. J. Virol. 63:1-8.
- Ruggero D and Pandolfi PP. (2003) Does the ribosome translate cancer? Nat. Rev. Cancer. 3:179-92.
- Schaad MC, Lellis AD, and Carrington JC. (1997) VPg of tobacco etch potyvirus is a host genotype-specific determinant for long-distance movement. J. Virol. 71:8624-8631.
- Shantz LM, Pegg AE. (1994) Overproduction of ornithine decarboxylase caused by relief of translational repression s associated with neoplastic transformation. Cancer Res. 54:2313-6.
- Sonenberg N, Gingras AC. (1998) The mRNA 5' cap-binding protein eIF4E and control of cell growth. Curr Opin Cell Biol. 10:268-75.
- Wittmann S, Chatel H, Fortin MG, Laliberte JF. (1997) Interaction of the viral protein genome linked of turnip mosaic potyvirus with the translational eukaryotic initiation factor (iso) 4E of Arabidopsis thaliana using the yeast two-hybrid system. Virology. 234:84-92.

## Claims

1. An isolated and purified protein comprising or consisting of the VPg protein of SEQ ID NO: 2 or a fragment of at least 15 contiguous amino acids of the protein consisting of SEQ ID NO: 2, having the ability to bind an eukaryotic initiation factor eIF4E, said protein and fragment thereof not comprising the amino acid motif YxxxxLØ, wherein x is any amino acid, Y is tyrosine (Tyr), L is leucine (Leu) and Ø is leucine (Leu), methionine (Met) or phenylalanine (Phe),
for use as a medicament.

2. The protein or fragment thereof for use according to claim 1, **characterized in that** they have the ability to bind a mammal initiation factor eIF4E.

3. The fragment of the VPg protein of SEQ ID NO: 2 for use according to claim 1, **characterized in that** said fragment is selected from the group consisting of SEQ ID NO: 14, SEQ ID NO: 16, SEQ ID NO: 18, SEQ ID NO: 20 and SEQ ID NO: 22.

4. The fragment of the VPg protein of SEQ ID NO: 2 for use according to claim 1, **characterized in that** it comprises or consists of at least 15, 20, 25 or 26 contiguous amino acids of SEQ ID NO: 16.

5. The protein or fragment thereof for use according to any of claims 1 to 4, for use as an anti-cancer agent.

6. The protein or fragment thereof according to any of claims 1 to 5 for use as an anti-cancer agent for treating cancer in which the cancer cells express the initiation factor eIF4E.

7. The protein or fragment thereof according to claim 6 for use as an anti-cancer agent for treating glioma, melanoma or a colon or lung cancer.

8. Method of screening *in vitro* for compounds which mimic the binding specificity of the VPg protein of SEQ ID NO: 2 with an isolated and purified initiation factor eIF4E of a subject, **characterized in that** it comprises the steps of i) contacting said eIF4E with a candidate compound and the protein or fragment thereof according to claims 1 to 4, and ii) detecting if the candidate compound inhibits the interaction between eIF4E and said protein or fragment thereof.

9. A recombinant vector expressing a nucleic acid encoding the protein or fragment thereof according to any of claims 1 to 4, for use as a medicament for preventing or treating cancer.

10. The recombinant vector for use according to claim 9, **characterized in that** the nucleic acid is selected from the group consisting of SEQ ID NO: 1, 13, 15, 17, 19 and 21.

11. A pharmaceutical composition comprising the protein or fragment thereof for use according to any of claims 1 to 4 or a recombinant vector for use according to claims 9 or 10, and a pharmaceutically acceptable carrier.

12. The pharmaceutical composition for use according to claim 11, **characterized in that** said pharmaceutically acceptable carrier is a cationic lipid.

13. The composition of claims 11 or 12 for use as a medicament for treating cancer, in which the initiation factor eIF4E is expressed.

## Patentansprüche

1. Isoliertes und gereinigtes Protein, umfassend das oder bestehend aus dem VPg-Protein gemäß SEQ ID NO: 2, oder ein/einem Fragment von mindestens 15 zusammenhängenden Aminosäuren des Proteins, welches aus SEQ ID NO: 2 besteht, welches die Fähigkeit besitzt, einen eukaryotischen Initiationsfaktor eIF4E zu binden, wobei das Protein und das Fragment davon nicht das Aminosäuremotiv YxxxxLØ umfassen, worin x jede beliebige Aminosäure ist, Y Thyrosin (Tyr) ist, L Leucin (Leu) ist, und 0 Leucin (Leu), Methionin (Met) oder Phenylalanin (Phe) ist, zur Verwendung als Medikament.

2. Protein oder Fragment davon zur Verwendung gemäß Anspruch 1, **dadurch gekennzeichnet, dass** sie die Fähigkeit besitzen, einen Säugetierinitiationsfaktor eIF4E zu binden.

3. Fragment des VPg-Proteins gemäß SEQ ID NO: 2 zur Verwendung gemäß Anspruch 1, **dadurch gekennzeichnet, dass** das Fragment ausgewählt ist aus der Gruppe bestehend aus SEQ ID NO: 14, SEQ ID NO: 16, SEQ ID NO: 18, SEQ ID NO: 20 und SEQ ID NO: 22.

4. Fragment des VPg-Proteins gemäß SEQ ID NO: 2 zur Verwendung gemäß Anspruch 1, **dadurch gekennzeichnet, dass** es mindestens 15, 20, 25 oder 26 zusammenhängende Aminosäuren von SEQ ID NO: 16 umfasst, oder daraus besteht.

5. Protein oder Fragment davon zur Verwendung gemäß einem der Ansprüche 1 bis 4 zur Verwendung als Anti-Krebs-Agens.

6. Protein oder Fragment davon gemäß einem der Ansprüche 1 bis 5 zur Verwendung als Anti-Krebs-Agens zur Behandlung von Krebs, bei dem die Krebszellen den Initiationsfaktor eIF4E exprimieren.

7. Protein oder Fragment davon gemäß Anspruch 6, zur Verwendung als Anti-Krebs-Agens zur Behandlung von Gliom, Melanom oder einem Dickdarm- oder Lungenkarzinom.

8. In-vitro-Screeningverfahren für Verbindungen, welche die Bindungsspezifität des VPg-Proteins gemäß SEQ ID NO: 2 mit einem isolierten und gereinigten Initiationsfaktor eIF4E eines Subjekts nachahmen, **dadurch gekennzeichnet, dass** es die Schritte von i) Inkontaktbringen des eIF4E mit einer Kandidatenverbindung und dem Protein oder Fragment davon gemäß Ansprüchen 1 bis 4, und ii) Detektieren, ob die Kandidatenverbindung die Wechselwirkung zwischen eIF4E und dem Protein oder Fragment davon inhibiert, umfasst.

9. Rekombinanter Vektor, welcher eine Nucleinsäure exprimiert, die das Protein oder Fragment davon gemäß einem der Ansprüche 1 bis 4 codiert, zur Verwendung als Medikament zur Prävention oder Behandlung von Krebs.

10. Rekombinanter Vektor zur Verwendung gemäß Anspruch 9, **dadurch gekennzeichnet, dass** die Nucleinsäure aus der Gruppe bestehend aus SEQ ID NO: 1, 13, 15, 17, 19 und 21 ausgewählt ist.

11. Pharmazeutische Zusammensetzung, umfassend das Protein oder Fragment davon, zur Verwendung gemäß einem der Ansprüche 1 bis 4, oder einen rekombinanten Vektor zur Verwendung gemäß Anspruch 9 oder 10, und einen pharmazeutisch annehmbaren Träger.

12. Pharmazeutische Zusammensetzung zur Verwendung gemäß Anspruch 11, **dadurch gekennzeichnet, dass** der pharmazeutisch annehmbare Träger ein kationisches Lipid ist.

13. Zusammensetzung gemäß Anspruch 11 oder 12, zur Verwendung als Medikament zur Behandlung von Krebs, bei dem der Initiationsfaktor eIF4E exprimiert wird.

## Revendications

1. Protéine isolée et purifiée comprenant, ou constituée de, la protéine VPg de SEQ ID NO : 2 ou un fragment de celle-ci d'au moins 15 acides aminés contigus de la protéine constituée de SEQ ID NO : 2, ayant la capacité de se lier à un facteur d'initiation eucaryote eIF4E, ladite protéine et ledit fragment de celle-ci ne comprenant pas le motif d'acides aminés YxxxxLØ, dans lequel x est tout acide aminé, Y est la tyrosine (Tyr), L est la leucine (Leu) et Ø est la leucine (Leu), la méthionine (Met) ou la phénylalanine (Phe),
pour son utilisation en tant que médicament.

2. Protéine ou fragment de celle-ci pour son utilisation selon la revendication 1, **caractérisés en ce qu'**ils ont la capacité de se lier à un facteur d'initiation eIF4E de mammifère.

3. Fragment de la protéine VPg de SEQ ID NO : 2 pour son utilisation selon la revendication 1, **caractérisé en ce que** ledit fragment est sélectionné dans le groupe constitué de SEQ ID NO : 14, SEQ ID NO : 16, SEQ ID NO : 18, SEQ ID NO : 20 et SEQ ID NO : 22.

4. Fragment de la protéine VPg de SEQ ID NO : 2 pour son utilisation selon la revendication 1, **caractérisé en ce qu'**il comprend au moins, ou est constitué d'au moins, 15, 20, 25 ou 26 acides aminés contigus de SEQ ID NO : 16.

5. Protéine ou fragment de celle-ci pour son utilisation selon l'une quelconque des revendications 1 à 4, pour son utilisation en tant qu'agent anti-cancéreux.

6. Protéine ou fragment de celle-ci selon l'une quelconque des revendications 1 à 5, pour son utilisation en tant qu'agent anti-cancéreux pour le traitement d'un cancer dans lequel les cellules cancéreuses expriment le facteur d'initiation eIF4E.

7. Protéine ou fragment de celle-ci selon la revendication 6, pour son utilisation en tant qu'agent anti-cancéreux pour le traitement d'un gliome, d'un mélanome ou d'un cancer du côlon ou du poumon.

8. Procédé de criblage *in vitro* de composés qui imitent la spécificité de liaison de la protéine VPg de SEQ ID NO : 2 avec un facteur d'initiation isolé et purifié eIF4E d'un sujet, **caractérisé en ce qu'**il comprend les étapes i) de mise en contact dudit eIF4E avec un composé candidat et la protéine ou le fragment de celle-ci selon les revendications 1 à 4, et ii) de détection du fait que le composé candidat inhibe ou non l'interaction entre eIF4E et ladite protéine ou ledit fragment de celle-ci.

9. Vecteur recombinant exprimant un acide nucléique codant pour la protéine ou un fragment de celle-ci selon l'une quelconque des revendications 1 à 4, pour son utilisation en tant que médicament pour la prévention ou le traitement du cancer.

10. Vecteur recombinant pour son utilisation selon la revendication 9, **caractérisé en ce que** l'acide nucléique est sélectionné dans le groupe constitué de SEQ ID NO : 1, 13, 15, 17, 19 et 21.

11. Composition pharmaceutique comprenant la protéine ou un fragment de celle-ci pour son utilisation selon l'une quelconque des revendications 1 à 4 ou un vecteur recombinant pour son utilisation selon la revendication 9 ou 10, et un véhicule pharmaceutiquement acceptable.

12. Composition pharmaceutique pour son utilisation selon la revendication 11, **caractérisée en ce que** ledit véhicule pharmaceutiquement acceptable est un lipide cationique.

13. Composition selon la revendication 11 ou 12, pour son utilisation en tant que médicament pour le traitement du cancer, dans lequel le facteur d'initiation eIF4E est exprimé.


### SEQUENCE LISTING

<110> CENTRE NATIONAL DE LA RECHERCHE SCIENTIFIQUE INSTITUTE OF BIOCHEMISTRY AND BIOPHYSICS - POLISH ACADEMY UNIVERSITE JOSEPH FOURIER CHROBOCZEK, Jadwiga ZAGORSKI, Wlodzimierz GRZELA, Renata
<120> Cell-proliferation inhibiting VPg proteins, fragments or analogs thereof and their applications
<130> BLO/XRN/clg-644/159
<160> 30
<170> PatentIn version 3.3
<210> 1
   <211> 564
   <212> DNA
   <213> Potato virus Y
<220>
   <221> CDS
   <222> (1)..(564)
<400> 1
   ggg aaa aat aaa tcc aag aga atc caa gcc ttg aag ttt cgc cat gct       48
   Gly Lys Asn Lys Ser Lys Arg Ile Gln Ala Leu Lys Phe Arg His Ala
   1               5                   10                  15
   cgt gac aaa agg gct ggc ttt gaa att gac aac aat gat gac aca ata       96
   Arg Asp Lys Arg Ala Gly Phe Glu Ile Asp Asn Asn Asp Asp Thr Ile
               20                  25                  30
   gag gaa ttc ttt gga tct gca tac agg aaa aag gga aaa ggt aaa ggt      144
   Glu Glu Phe Phe Gly Ser Ala Tyr Arg Lys Lys Gly Lys Gly Lys Gly
           35                  40                  45
   acc aca gtt ggt atg ggc aag tca agc agg agg ttc att aac atg tac      192
   Thr Thr Val Gly Met Gly Lys Ser Ser Arg Arg Phe Ile Asn Met Tyr
       50                  55                  60
   ggg ttc gac cca aca gag tac tca ttc atc caa ttc gtt gat cca ctc      240
   Gly Phe Asp Pro Thr Glu Tyr Ser Phe Ile Gln Phe Val Asp Pro Leu
   65                  70                  75                  80
   act ggg gca caa ata gaa gag aat gtc tat gct gac att aga gat att      288
   Thr Gly Ala Gln Ile Glu Glu Asn Val Tyr Ala Asp Ile Arg Asp Ile
                   85                  90                  95
   caa gag aga ttt agt gaa gtg cga aag aaa atg gtt gag aat gat gac      336
   Gln Glu Arg Phe Ser Glu Val Arg Lys Lys Met Val Glu Asn Asp Asp
               100                 105                 110
   att gaa atg caa gcc ttg ggt agt aac acg acc ata cat gca tac ttt      384
   Ile Glu Met Gln Ala Leu Gly Ser Asn Thr Thr Ile His Ala Tyr Phe
           115                 120                 125
   agg aaa gat tgg tct gac aaa gct ttg aag att gat tta atg cca cat      432
   Arg Lys Asp Trp Ser Asp Lys Ala Leu Lys Ile Asp Leu Met Pro His
       130                 135                 140
   aac cca ctc aaa gtt tgt gac aaa aca aat ggc att gcc aaa ttt cct      480
   Asn Pro Leu Lys Val Cys Asp Lys Thr Asn Gly Ile Ala Lys Phe Pro
   145                 150                 155                 160
   gag aga gag ctc gaa cta agg cag act ggg cca gct gta gaa gtc gac      528
   Glu Arg Glu Leu Glu Leu Arg Gln Thr Gly Pro Ala Val Glu Val Asp
                   165                 170                 175
   gtg aag gac ata cca gcg cag gag gtg gag cat gaa                      564
   Val Lys Asp Ile Pro Ala Gln Glu Val Glu His Glu
               180                 185
<210> 2
   <211> 188
   <212> PRT
   <213> Potato virus Y
<400> 2
   Gly Lys Asn Lys Ser Lys Arg Ile Gln Ala Leu Lys Phe Arg His Ala
   1               5                   10                  15
   Arg Asp Lys Arg Ala Gly Phe Glu Ile Asp Asn Asn Asp Asp Thr Ile
               20                  25                  30
   Glu Glu Phe Phe Gly Ser Ala Tyr Arg Lys Lys Gly Lys Gly Lys Gly
           35                  40                  45
   Thr Thr Val Gly Met Gly Lys Ser Ser Arg Arg Phe Ile Asn Met Tyr
       50                  55                  60
   Gly Phe Asp Pro Thr Glu Tyr Ser Phe Ile Gln Phe Val Asp Pro Leu
   65                  70                  75                  80
   Thr Gly Ala Gln Ile Glu Glu Asn Val Tyr Ala Asp Ile Arg Asp Ile
                   85                  90                  95
   Gln Glu Arg Phe Ser Glu Val Arg Lys Lys Met Val Glu Asn Asp Asp
               100                 105                 110
   Ile Glu Met Gln Ala Leu Gly Ser Asn Thr Thr Ile His Ala Tyr Phe
           115                 120                 125
   Arg Lys Asp Trp Ser Asp Lys Ala Leu Lys Ile Asp Leu Met Pro His
       130                 135                 140
   Asn Pro Leu Lys Val Cys Asp Lys Thr Asn Gly Ile Ala Lys Phe Pro
   145                 150                 155                 160
   Glu Arg Glu Leu Glu Leu Arg Gln Thr Gly Pro Ala Val Glu Val Asp
                   165                 170                 175
   Val Lys Asp Ile Pro Ala Gln Glu Val Glu His Glu
               180                 185
<210> 3
   <211> 564
   <212> DNA
   <213> Tobacco etch virus
<220>
   <221> CDS
   <222> (1)..(564)
<400> 3
   ggg aag aag aat cag aag cac aag ctt aag atg aga gag gcg cgt ggg       48
   Gly Lys Lys Asn Gln Lys His Lys Leu Lys Met Arg Glu Ala Arg Gly
   1               5                   10                  15
   gct aga ggg caa tat gag gtt gca gcg gag cca gag gcg cta gaa cat       96
   Ala Arg Gly Gln Tyr Glu Val Ala Ala Glu Pro Glu Ala Leu Glu His
               20                  25                  30
   tac ttt gga agc gca tat aat aac aaa gga aag cgc aag ggc acc acg      144
   Tyr Phe Gly Ser Ala Tyr Asn Asn Lys Gly Lys Arg Lys Gly Thr Thr
           35                  40                  45
   aga gga atg ggt gca aag tct cgg aaa ttc ata aac atg tat ggg ttt      192
   Arg Gly Met Gly Ala Lys Ser Arg Lys Phe Ile Asn Met Tyr Gly Phe
       50                  55                  60
   gat cca act gat ttt tca tac att agg ttt gtg gat cca ttg aca ggt      240
   Asp Pro Thr Asp Phe Ser Tyr Ile Arg Phe Val Asp Pro Leu Thr Gly
   65                  70                  75                  80
   cac act att gat gag tcc aca aac gca cct att gat tta gtg cag cat      288
   His Thr Ile Asp Glu Ser Thr Asn Ala Pro Ile Asp Leu Val Gln His
                   85                  90                  95
   gag ttt gga aag gtt aga aca cgc atg tta att gac gat gag ata gag      336
   Glu Phe Gly Lys Val Arg Thr Arg Met Leu Ile Asp Asp Glu Ile Glu
               100                 105                 110
   cct caa agt ctt agc acc cac acc aca atc cat gct tat ttg gtg aat      384
   Pro Gln Ser Leu Ser Thr His Thr Thr Ile His Ala Tyr Leu Val Asn
           115                 120                 125
   agt ggc acg aag aaa gtt ctt aag gtt gat tta aca cca cac tcg tcg      432
   Ser Gly Thr Lys Lys Val Leu Lys Val Asp Leu Thr Pro His Ser Ser
       130                 135                 140
   cta cgt gcg agt gag aaa tca aca gca ata atg gga ttt cct gaa agg      480
   Leu Arg Ala Ser Glu Lys Ser Thr Ala Ile Met Gly Phe Pro Glu Arg
   145                 150                 155                 160
   gag aat gaa ttg cgt caa acc ggc atg gca gtg cca gtg gct tat gat      528
   Glu Asn Glu Leu Arg Gln Thr Gly Met Ala Val Pro Val Ala Tyr Asp
                   165                 170                 175
   caa ttg cca cca aag aat gag gac ttg acg ttt gaa                      564
   Gln Leu Pro Pro Lys Asn Glu Asp Leu Thr Phe Glu
               180                 185
<210> 4
   <211> 188
   <212> PRT
   <213> Tobacco etch virus
<400> 4
   Gly Lys Lys Asn Gln Lys His Lys Leu Lys Met Arg Glu Ala Arg Gly
   1               5                   10                  15
   Ala Arg Gly Gln Tyr Glu Val Ala Ala Glu Pro Glu Ala Leu Glu His
   20 25 30
   Tyr Phe Gly Ser Ala Tyr Asn Asn Lys Gly Lys Arg Lys Gly Thr Thr
   35 40 45
   Arg Gly Met Gly Ala Lys Ser Arg Lys Phe Ile Asn Met Tyr Gly Phe
   50 55 60
   Asp Pro Thr Asp Phe Ser Tyr Ile Arg Phe Val Asp Pro Leu Thr Gly
   65 70 75 80
   His Thr Ile Asp Glu Ser Thr Asn Ala Pro Ile Asp Leu Val Gln His
   85 90 95
   Glu Phe Gly Lys Val Arg Thr Arg Met Leu Ile Asp Asp Glu Ile Glu
   100 105 110
   Pro Gln Ser Leu Ser Thr His Thr Thr Ile His Ala Tyr Leu Val Asn
   115 120 125
   Ser Gly Thr Lys Lys Val Leu Lys Val Asp Leu Thr Pro His Ser Ser
   130 135 140
   Leu Arg Ala Ser Glu Lys Ser Thr Ala Ile Met Gly Phe Pro Glu Arg
   145 150 155 160
   Glu Asn Glu Leu Arg Gln Thr Gly Met Ala Val Pro Val Ala Tyr Asp
   165 170 175
   Gln Leu Pro Pro Lys Asn Glu Asp Leu Thr Phe Glu
   180 185
<210> 5
   <211> 573
   <212> DNA
   <213> Clover yellow vein virus
<220>
   <221> CDS
   <222> (1)..(573)
<400> 5
   ggg aaa agt aag aga aca aga caa aag ttg aag ttc aga gcg gca aga      48
   Gly Lys Ser Lys Arg Thr Arg Gln Lys Leu Lys Phe Arg Ala Ala Arg
   1               5                   10                  15
   gac atg aag gat cgt tat gaa gtg cat gcc gat gag ggg act tta gtg      96
   Asp Met Lys Asp Arg Tyr Glu Val His Ala Asp Glu Gly Thr Leu Val
               20                  25                  30
   gaa aat ttt gga act cgt tat tca aag aaa ggc aag aca aaa ggt act      144
   Glu Asn Phe Gly Thr Arg Tyr Ser Lys Lys Gly Lys Thr Lys Gly Thr
           35                  40                  45
   gtt gtg ggt ttg ggt gca aaa aca aga cgg ttc act aac atg tat ggt      192
   Val Val Gly Leu Gly Ala Lys Thr Arg Arg Phe Thr Asn Met Tyr Gly
       50                  55                  60
   ttt gac ccc acg gag tat tca ttt gct agg tat ctt gat cca atc acg      240
   Phe Asp Pro Thr Glu Tyr Ser Phe Ala Arg Tyr Leu Asp Pro Ile Thr
   65                  70                  75                  80
   ggt gca aca ttg gat gaa acc cca att cac aat gta aat ttg gtt gct      288
   Gly Ala Thr Leu Asp Glu Thr Pro Ile His Asn Val Asn Leu Val Ala
                   85                  90                  95
   gag cat ttt ggc gac ata agg ctt gat atg gtt gac aag gag tta ctt      336
   Glu His Phe Gly Asp Ile Arg Leu Asp Met Val Asp Lys Glu Leu Leu
               100                 105                 110
   gac aaa cag cac tta tac ctc aag aga cca ata gaa tgt tac ttt gta      384
   Asp Lys Gln His Leu Tyr Leu Lys Arg Pro Ile Glu Cys Tyr Phe Val
           115                 120                 125
   aag gat gct ggt cag aag gtg atg agg att gat cta aca ccc cac aac      432
   Lys Asp Ala Gly Gln Lys Val Met Arg Ile Asp Leu Thr Pro His Asn
       130                 135                 140
   cca ttg ttg gca agc gat gtt agc aca acc ata atg ggt tat cct gag      480
   Pro Leu Leu Ala Ser Asp Val Ser Thr Thr Ile Met Gly Tyr Pro Glu
   145                 150                 155                 160
   aga gaa ggt gaa ctc cgt caa act gga aag gca agg tta gtc gac cca      528
   Arg Glu Gly Glu Leu Arg Gln Thr Gly Lys Ala Arg Leu Val Asp Pro
                   165                 170                 175
   tca gag ttg ccc gcg cgg aat gag gat att gat gca gag ttt gag          573
   Ser Glu Leu Pro Ala Arg Asn Glu Asp Ile Asp Ala Glu Phe Glu
               180                 185                 190
<210> 6
   <211> 191
   <212> PRT
   <213> clover yellow vein virus
<400> 6
   Gly Lys Ser Lys Arg Thr Arg Gln Lys Leu Lys Phe Arg Ala Ala Arg
   1               5                   10                  15
   Asp Met Lys Asp Arg Tyr Glu Val His Ala Asp Glu Gly Thr Leu Val
               20                  25                  30
   Glu Asn Phe Gly Thr Arg Tyr Ser Lys Lys Gly Lys Thr Lys Gly Thr
           35                  40                  45
   Val Val Gly Leu Gly Ala Lys Thr Arg Arg Phe Thr Asn Met Tyr Gly
       50                  55                  60
   Phe Asp Pro Thr Glu Tyr Ser Phe Ala Arg Tyr Leu Asp Pro Ile Thr
   65                  70                  75                  80
   Gly Ala Thr Leu Asp Glu Thr Pro Ile His Asn Val Asn Leu Val Ala
                   85                  90                  95
   Glu His Phe Gly Asp Ile Arg Leu Asp Met Val Asp Lys Glu Leu Leu
               100                 105                 110
   Asp Lys Gln His Leu Tyr Leu Lys Arg Pro Ile Glu Cys Tyr Phe Val
           115                 120                 125
   Lys Asp Ala Gly Gln Lys Val Met Arg Ile Asp Leu Thr Pro His Asn
       130                 135                 140
   Pro Leu Leu Ala Ser Asp Val Ser Thr Thr Ile Met Gly Tyr Pro Glu
   145                 150                 155                 160
   Arg Glu Gly Glu Leu Arg Gln Thr Gly Lys Ala Arg Leu Val Asp Pro
                   165                 170                 175
   Ser Glu Leu Pro Ala Arg Asn Glu Asp Ile Asp Ala Glu Phe Glu
               180                 185                 190
<210> 7
   <211> 549
   <212> DNA
   <213> Tobacco vein mottling virus
<220>
   <221> CDS
   <222> (1)..(549)
<400> 7
   ggc aag agt aga cgc cga ctt caa ttc aga aaa gca agg gac gat aag      48
   Gly Lys Ser Arg Arg Arg Leu Gln Phe Arg Lys Ala Arg Asp Asp Lys
   1               5                   10                  15
   atg ggt tat atc atg cat ggt gaa ggg gac aca att gaa cat ttc ttt      96
   Met Gly Tyr Ile Met His Gly Glu Gly Asp Thr Ile Glu His Phe Phe
               20                  25                  30
   ggc gcg gcc tat aca aag aaa gga aag tcc aaa ggg aaa act cat ggc      144
   Gly Ala Ala Tyr Thr Lys Lys Gly Lys Ser Lys Gly Lys Thr His Gly
           35                  40                  45
   gct gga acg aag gcg cac aaa ttt gtg aat atg tat gga gtc agt cct      192
   Ala Gly Thr Lys Ala His Lys Phe Val Asn Met Tyr Gly Val Ser Pro
       50                  55                  60
   gat gaa tat tca tat gta cgt tat cta gat cca gtc acg ggt gcg act      240
   Asp Glu Tyr Ser Tyr Val Arg Tyr Leu Asp Pro Val Thr Gly Ala Thr
   65                  70                  75                  80
   cta gat gaa tct cca atg aca gac tta aac att gtg caa gaa cac ttt      288
   Leu Asp Glu Ser Pro Met Thr Asp Leu Asn Ile Val Gln Glu His Phe
                   85                  90                  95
   gga gaa atc agg agg gaa gct ata ctt gct gat gca atg tca cca caa      336
   Gly Glu Ile Arg Arg Glu Ala Ile Leu Ala Asp Ala Met Ser Pro Gln
               100                 105                 110
   caa agg aac aag gga atc cag gca tac ttt gtt aga aat tca aca atg      384
   Gln Arg Asn Lys Gly Ile Gln Ala Tyr Phe Val Arg Asn Ser Thr Met
           115                 120                 125
   cca att ctc aaa gtt gat ctg aca cca cac att cct ctt aaa gta tgt      432
   Pro Ile Leu Lys Val Asp Leu Thr Pro His Ile Pro Leu Lys Val Cys
       130                 135                 140
   gag tct aat aat att gct ggc ttt cca gag aga gaa ggg gaa ttg cgg      480
   Glu Ser Asn Asn Ile Ala Gly Phe Pro Glu Arg Glu Gly Glu Leu Arg
   145                 150                 155                 160
   aga aca ggc cca aca gaa aca ctc ccc ttt gat gca ctg ccc cca gaa      528
   Arg Thr Gly Pro Thr Glu Thr Leu Pro Phe Asp Ala Leu Pro Pro Glu
                   165                 170                 175
   aaa caa gaa gtt gca ttc gag                                          549
   Lys Gln Glu Val Ala Phe Glu
               180
<210> 8
   <211> 183
   <212> PRT
   <213> Tobacco vein mottling virus
<400> 8
   Gly Lys Ser Arg Arg Arg Leu Gln Phe Arg Lys Ala Arg Asp Asp Lys
   1               5                   10                  15
   Met Gly Tyr Ile Met His Gly Glu Gly Asp Thr Ile Glu His Phe Phe
               20                  25                  30
   Gly Ala Ala Tyr Thr Lys Lys Gly Lys Ser Lys Gly Lys Thr His Gly
           35                  40                  45
   Ala Gly Thr Lys Ala His Lys Phe Val Asn Met Tyr Gly Val Ser Pro
       50                  55                  60
   Asp Glu Tyr Ser Tyr Val Arg Tyr Leu Asp Pro Val Thr Gly Ala Thr
   65                  70                  75                  80
   Leu Asp Glu Ser Pro Met Thr Asp Leu Asn Ile Val Gln Glu His Phe
                   85                  90                  95
   Gly Glu Ile Arg Arg Glu Ala Ile Leu Ala Asp Ala Met Ser Pro Gln
               100                 105                 110
   Gln Arg Asn Lys Gly Ile Gln Ala Tyr Phe Val Arg Asn Ser Thr Met
           115                 120                 125
   Pro Ile Leu Lys Val Asp Leu Thr Pro His Ile Pro Leu Lys Val Cys
       130                 135                 140
   Glu Ser Asn Asn Ile Ala Gly Phe Pro Glu Arg Glu Gly Glu Leu Arg
   145                 150                 155                 160
   Arg Thr Gly Pro Thr Glu Thr Leu Pro Phe Asp Ala Leu Pro Pro Glu
                   165                 170                 175
   Lys Gln Glu Val Ala Phe Glu
               180
<210> 9
   <211> 576
   <212> DNA
   <213> Turnip mosaic virus
<220>
   <221> CDS
   <222> (1)..(576)
<400> 9
   gct aaa ggc aag agg caa aga cag aaa ctg aag ttt cgc aac gcc cga      48
   Ala Lys Gly Lys Arg Gln Arg Gln Lys Leu Lys Phe Arg Asn Ala Arg
   1               5                   10                  15
   gac aac aaa atg ggt agg gaa gtg tat gga gac gac gac acc ata gag      96
   Asp Asn Lys Met Gly Arg Glu Val Tyr Gly Asp Asp Asp Thr Ile Glu
               20                  25                  30
   cat ttc ttt gga gat gct tac aca aag aaa gga aag agc aaa ggc agg     144
   His Phe Phe Gly Asp Ala Tyr Thr Lys Lys Gly Lys Ser Lys Gly Arg
           35                  40                  45
   aca cgt ggc atc ggg cat aaa aac agg aag ttc atc aat atg tat ggg     192
   Thr Arg Gly Ile Gly His Lys Asn Arg Lys Phe Ile Asn Met Tyr Gly
       50                  55                  60
   ttt gat cct gaa gat ttc tct gca gtt agg ttc gta gat cca ctc aca     240
   Phe Asp Pro Glu Asp Phe Ser Ala Val Arg Phe Val Asp Pro Leu Thr
   65                  70                  75                  80
   ggg gcg aca ata gat gaa aac cca ttc acg gac atc act ctt gtg caa     288
   Gly Ala Thr Ile Asp Glu Asn Pro Phe Thr Asp Ile Thr Leu Val Gln
                   85                  90                  95
   aag cac ttt ggt gac ata aga atg gat ttg ctc gga gag gat gag ctg     336
   Lys His Phe Gly Asp Ile Arg Met Asp Leu Leu Gly Glu Asp Glu Leu
               100                 105                 110
   gac cca aat gag ata cga atg aac agg gca atc cag gcc tac tac atg     384
   Asp Pro Asn Glu Ile Arg Met Asn Arg Ala Ile Gln Ala Tyr Tyr Met
           115                 120                 125
   aac aat aaa aca ggc aag gct ctg aag gta gac ttg aca cca cac ata     432
   Asn Asn Lys Thr Gly Lys Ala Leu Lys Val Asp Leu Thr Pro His Ile
       130                 135                 140
   cct ctc aag gtg tgt gac ctt cat gca acc att gct gga ttc cca gag     480
   Pro Leu Lys Val Cys Asp Leu His Ala Thr Ile Ala Gly Phe Pro Glu
   145                 150                 155                 160
   aga gag aat gaa ctg agg caa act gga aag gct cag cct atc agt ata     528
   Arg Glu Asn Glu Leu Arg Gln Thr Gly Lys Ala Gln Pro Ile Ser Ile
                   165                 170                 175
   gat gaa gtg ccg aga gcc aac aac gaa ctc atc cca gtg gac cat gag     576
   Asp Glu Val Pro Arg Ala Asn Asn Glu Leu Ile Pro Val Asp His Glu
               180                 185                 190
<210> 10
   <211> 192
   <212> PRT
   <213> Turnip mosaic virus
<400> 10
   Ala Lys Gly Lys Arg Gln Arg Gln Lys Leu Lys Phe Arg Asn Ala Arg
   1               5                   10                  15
   Asp Asn Lys Met Gly Arg Glu Val Tyr Gly Asp Asp Asp Thr Ile Glu
               20                  25                  30
   His Phe Phe Gly Asp Ala Tyr Thr Lys Lys Gly Lys Ser Lys Gly Arg
           35                  40                  45
   Thr Arg Gly Ile Gly His Lys Asn Arg Lys Phe Ile Asn Met Tyr Gly
       50                  55                  60
   Phe Asp Pro Glu Asp Phe Ser Ala Val Arg Phe Val Asp Pro Leu Thr
   65                  70                  75                  80
   Gly Ala Thr Ile Asp Glu Asn Pro Phe Thr Asp Ile Thr Leu Val Gln
                   85                  90                  95
   Lys His Phe Gly Asp Ile Arg Met Asp Leu Leu Gly Glu Asp Glu Leu
               100                 105                 110
   Asp Pro Asn Glu Ile Arg Met Asn Arg Ala Ile Gln Ala Tyr Tyr Met
           115                 120                 125
   Asn Asn Lys Thr Gly Lys Ala Leu Lys Val Asp Leu Thr Pro His Ile
       130                 135                 140
   Pro Leu Lys Val Cys Asp Leu His Ala Thr Ile Ala Gly Phe Pro Glu
   145                 150                 155                 160
   Arg Glu Asn Glu Leu Arg Gln Thr Gly Lys Ala Gln Pro Ile Ser Ile
                   165                 170                 175
   Asp Glu Val Pro Arg Ala Asn Asn Glu Leu Ile Pro Val Asp His Glu
               180                 185                 190
<210> 11
   <211> 579
   <212> DNA
   <213> Lettuce mosaic virus
<220>
   <221> CDS
   <222> (1)..(579)
<400> 11
   ggc aaa ggt aaa cgg caa aga cag aag ctt cgt tat cga caa gca agg      48
   Gly Lys Gly Lys Arg Gln Arg Gln Lys Leu Arg Tyr Arg Gln Ala Arg
   1               5                   10                  15
   gat aat aag atg ggc att gaa gtt tat ggt gac gat gcg acg atg gaa      96
   Asp Asn Lys Met Gly Ile Glu Val Tyr Gly Asp Asp Ala Thr Met Glu
               20                  25                  30
   cac tat ttt gga gct gcg tac aca gag aaa gga aag aaa tcc gga aag      144
   His Tyr Phe Gly Ala Ala Tyr Thr Glu Lys Gly Lys Lys Ser Gly Lys
           35                  40                  45
   acg aaa gga atg ggg acg aaa aat cga aga ttt gtt aac atg tat ggg      192
   Thr Lys Gly Met Gly Thr Lys Asn Arg Arg Phe Val Asn Met Tyr Gly
       50                  55                  60
   tac aac cca gaa gat tac tcg ttc atc cga ttt ctg gac cca ctc aca      240
   Tyr Asn Pro Glu Asp Tyr Ser Phe Ile Arg Phe Leu Asp Pro Leu Thr
   65                  70                  75                  80
   ggg aaa aca atg gat gaa caa gta ttc act gac ata agt ctc gtc caa      288
   Gly Lys Thr Met Asp Glu Gln Val Phe Thr Asp Ile Ser Leu Val Gln
                   85                  90                  95
   gat gcc ttt ggt aaa gaa aga ctc aaa ctc ctg tcc gaa ggg gaa att      336
   Asp Ala Phe Gly Lys Glu Arg Leu Lys Leu Leu Ser Glu Gly Glu Ile
               100                 105                 110
   gag tca gag cac atg cga aat ggg att aga gct tat ctt gtc aag aat      384
   Glu Ser Glu His Met Arg Asn Gly Ile Arg Ala Tyr Leu Val Lys Asn
           115                 120                 125
   ctt acc aca gca gct ctc gaa ata gac atg acc cct cac aac tct tgc      432
   Leu Thr Thr Ala Ala Leu Glu Ile Asp Met Thr Pro His Asn Ser Cys
       130                 135                 140
   cag ctc gga acc aag aca aac aac ata gca gga ttt gta gac agg gag      480
   Gln Leu Gly Thr Lys Thr Asn Asn Ile Ala Gly Phe Val Asp Arg Glu
   145                 150                 155                 160
   tac gaa ttg cgt caa acc ggg gaa gcc agg gtt gtt gct cca gca ctg      528
   Tyr Glu Leu Arg Gln Thr Gly Glu Ala Arg Val Val Ala Pro Ala Leu
                   165                 170                 175
   att cca aaa gac aat cca atc acg gac gag gat att ccc gta aag cat      576
   Ile Pro Lys Asp Asn Pro Ile Thr Asp Glu Asp Ile Pro Val Lys His
               180                 185                 190
   gaa                                                                  579
   Glu
<210> 12
   <211> 193
   <212> PRT
   <213> Lettuce mosaic virus
<400> 12
   Gly Lys Gly Lys Arg Gln Arg Gln Lys Leu Arg Tyr Arg Gln Ala Arg
   1               5                   10                  15
   Asp Asn Lys Met Gly Ile Glu Val Tyr Gly Asp Asp Ala Thr Met Glu
               20                  25                  30
   His Tyr Phe Gly Ala Ala Tyr Thr Glu Lys Gly Lys Lys Ser Gly Lys
           35                  40                  45
   Thr Lys Gly Met Gly Thr Lys Asn Arg Arg Phe Val Asn Met Tyr Gly
       50                  55                  60
   Tyr Asn Pro Glu Asp Tyr Ser Phe Ile Arg Phe Leu Asp Pro Leu Thr
   65                  70                  75                  80
   Gly Lys Thr Met Asp Glu Gln Val Phe Thr Asp Ile Ser Leu Val Gln
                   85                  90                  95
   Asp Ala Phe Gly Lys Glu Arg Leu Lys Leu Leu Ser Glu Gly Glu Ile
               100                 105                 110
   Glu Ser Glu His Met Arg Asn Gly Ile Arg Ala Tyr Leu Val Lys Asn
           115                 120                 125
   Leu Thr Thr Ala Ala Leu Glu Ile Asp Met Thr Pro His Asn Ser Cys
       130                 135                 140
   Gln Leu Gly Thr Lys Thr Asn Asn Ile Ala Gly Phe Val Asp Arg Glu
   145                 150                 155                 160
   Tyr Glu Leu Arg Gln Thr Gly Glu Ala Arg Val Val Ala Pro Ala Leu
                   165                 170                 175
   Ile Pro Lys Asp Asn Pro Ile Thr Asp Glu Asp Ile Pro Val Lys His
               180                 185                 190
   Glu
<210> 13
   <211> 162
   <212> DNA
   <213> Potato virus Y
<220>
   <221> CDS
   <222> (1) .. (162)
<400> 13
   agg aaa aag gga aaa ggt aaa ggt acc aca gtt ggt atg ggc aag tca      48
   Arg Lys Lys Gly Lys Gly Lys Gly Thr Thr Val Gly Met Gly Lys Ser
   1               5                   10                  15
   agc agg agg ttc att aac atg tac ggg ttc gac cca aca gag tac tca      96
   Ser Arg Arg Phe Ile Asn Met Tyr Gly Phe Asp Pro Thr Glu Tyr Ser
               20                  25                  30
   ttc atc caa ttc gtt gat cca ctc act ggg gca caa ata gaa gag aat     144
   Phe Ile Gln Phe Val Asp Pro Leu Thr Gly Ala Gln Ile Glu Glu Asn
           35                  40                  45
   gtc tat gct gac att aga                                             162
   Val Tyr Ala Asp Ile Arg
       50
<210> 14
   <211> 54
   <212> PRT
   <213> Potato virus Y
<400> 14
   Arg Lys Lys Gly Lys Gly Lys Gly Thr Thr Val Gly Met Gly Lys Ser
   1               5                   10                  15
   Ser Arg Arg Phe Ile Asn Met Tyr Gly Phe Asp Pro Thr Glu Tyr Ser
               20                  25                  30
   Phe Ile Gln Phe Val Asp Pro Leu Thr Gly Ala Gln Ile Glu Glu Asn
           35                  40                  45
   Val Tyr Ala Asp Ile Arg
       50
<210> 15
   <211> 126
   <212> DNA
   <213> Potato virus Y
<220>
   <221> CDS
   <222> (1)..(126)
<400> 15
   agg aaa aag gga aaa ggt aaa ggt acc aca gtt ggt atg ggc aag tca      48
   Arg Lys Lys Gly Lys Gly Lys Gly Thr Thr Val Gly Met Gly Lys Ser
   1               5                   10                  15
   agc agg agg ttc att aac atg tac ggg ttc gac cca aca gag tac tca      96
   Ser Arg Arg Phe Ile Asn Met Tyr Gly Phe Asp Pro Thr Glu Tyr Ser
               20                  25                  30
   ttc atc caa ttc gtt gat cca ctc act ggg                              126
   Phe Ile Gln Phe Val Asp Pro Leu Thr Gly
           35                  40
<210> 16
   <211> 42
   <212> PRT
   <213> Potato virus Y
<400> 16
   Arg Lys Lys Gly Lys Gly Lys Gly Thr Thr Val Gly Met Gly Lys Ser
   1               5                   10                  15
   Ser Arg Arg Phe Ile Asn Met Tyr Gly Phe Asp Pro Thr Glu Tyr Ser
               20                  25                  30
   Phe Ile Gln Phe Val Asp Pro Leu Thr Gly
           35                  40
<210> 17
   <211> 78
   <212> DNA
   <213> Potato virus Y
<220>
   <221> CDS
   <222> (1)..(78)
<400> 17
   agg aaa aag gga aaa ggt aaa ggt acc aca gtt ggt atg ggc aag tca      48
   Arg Lys Lys Gly Lys Gly Lys Gly Thr Thr Val Gly Met Gly Lys Ser
   1               5                   10                  15
   agc agg agg ttc att aac atg tac ggg ttc                              78
   Ser Arg Arg Phe Ile Asn Met Tyr Gly Phe
               20                  25
<210> 18
   <211> 26
   <212> PRT
   <213> Potato virus Y
<400> 18
   Arg Lys Lys Gly Lys Gly Lys Gly Thr Thr Val Gly Met Gly Lys Ser
   1               5                   10                  15
   Ser Arg Arg Phe Ile Asn Met Tyr Gly Phe
               20                  25
<210> 19
   <211> 57
   <212> DNA
   <213> Potato virus Y
<220>
   <221> CDS
   <222> (1)..(57)
<400> 19
   agg aaa aag gga aaa ggt aaa ggt acc aca gtt ggt atg ggc aag tca      48
   Arg Lys Lys Gly Lys Gly Lys Gly Thr Thr Val Gly Met Gly Lys Ser
   1               5                   10                  15
   agc agg agg                                                          57
   Ser Arg Arg
<210> 20
   <211> 19
   <212> PRT
   <213> Potato virus Y
<400> 20
   Arg Lys Lys Gly Lys Gly Lys Gly Thr Thr Val Gly Met Gly Lys Ser
   1               5                   10                  15
   Ser Arg Arg
<210> 21
   <211> 105
   <212> DNA
   <213> Potato virus Y
<220>
   <221> CDS
   <222> (1)..(105)
<400> 21
   ttc att aac atg tac ggg ttc gac cca aca gag tac tca ttc atc caa       48
   Phe Ile Asn Met Tyr Gly Phe Asp Pro Thr Glu Tyr Ser Phe Ile Gln
   1               5                   10                  15
   ttc gtt gat cca ctc act ggg gca caa ata gaa gag aat gtc tat gct       96
   Phe Val Asp Pro Leu Thr Gly Ala Gln Ile Glu Glu Asn Val Tyr Ala
               20                  25                  30
   gac att aga                                                           105
   Asp Ile Arg
           35
<210> 22
   <211> 35
   <212> PRT
   <213> Potato virus Y
<400> 22
   Phe Ile Asn Met Tyr Gly Phe Asp Pro Thr Glu Tyr Ser Phe Ile Gln
   1               5                   10                  15
   Phe Val Asp Pro Leu Thr Gly Ala Gln Ile Glu Glu Asn Val Tyr Ala
               20                  25                  30
   Asp Ile Arg
           35
<210> 23
   <211> 25
   <212> DNA
   <213> Artificial
<220>
   <223> VPg gene PCR primer
<400> 23
   gggggggatc catggggaaa ataaa 25
<210> 24
   <211> 27
   <212> DNA
   <213> Artificial
<220>
   <223> VPg gene PCR primer
<400> 24
   cccccagatc tctattattc atgctcc 27
<210> 25
   <211> 129
   <212> DNA
   <213> Autographa californica nucleopolyhedrovirus
<400> 25
   atcatggaga taattaaaat gataaccatc tcgcaaataa ataagtattt tactgttttc 60
   gtaacagttt tgtaataaaa aaacctataa atattccgga ttattcatac cgtcccacca 120
   tcgggcgcg 129
<210> 26
   <211> 4
   <212> PRT
   <213> Artificial
<220>
   <223> VPg motif
<400> 26
   Asn Met Tyr Gly
   1
<210> 27
   <211> 111
   <212> PRT
   <213> Feline calicivirus
<400> 27
   Ala Lys Gly Lys Thr Lys Ser Lys Val Gly Pro Tyr Arg Gly Arg Gly
   1               5                   10                  15
   Val Ala Leu Thr Asp Asp Glu Tyr Asp Glu Trp Arg Glu His Asn Ala
               20                  25                  30
   Thr Arg Lys Leu Asp Leu Ser Val Glu Asp Phe Leu Met Leu Arg His
           35                  40                  45
   Arg Ala Ala Leu Gly Ala Asp Asp Ala Asp Ala Val Lys Phe Arg Ser
       50                  55                  60
   Trp Trp Asn Ser Arg Ser Arg Leu Ala Asp Asp Tyr Glu Asp Val Thr
   65                  70                  75                  80
   Val Ile Gly Lys Gly Gly Val Lys His Glu Lys Ile Arg Thr Asn Thr
                   85                  90                  95
   Leu Arg Ala Val Asp Arg Gly Tyr Asp Val Ser Phe Ala Glu Glu
               100                 105                 110
<210> 28
   <211> 132
   <212> PRT
   <213> Human calicivirus
<400> 28
   Gly Lys Lys Gly Lys Asn Lys Thr Gly Arg Gly Lys Lys His Thr Ala
   1               5                   10                  15
   Phe Ser Ser Lys Gly Leu Ser Asp Glu Glu Tyr Asp Glu Tyr Lys Arg
   20 25 30
   Ile Arg Glu Glu Arg Asn Gly Lys Tyr Ser Ile Glu Glu Tyr Leu Gln
   35 40 45
   Asp Arg Asp Lys Tyr Tyr Glu Glu Val Ala Ile Ala Arg Ala Thr Glu
   50 55 60
   Glu Asp Phe Cys Glu Glu Glu Glu Ala Lys Ile Arg Gln Arg Ile Phe
   65 70 75 80
   Arg Pro Thr Arg Lys Gln Arg Lys Glu Glu Arg Ala Ser Leu Gly Leu
   85 90 95
   Val Thr Gly Ser Glu Ile Arg Lys Arg Asn Pro Asp Asp Phe Lys Pro
   100 105 110
   Lys Gly Lys Leu Trp Ala Asp Asp Asp Arg Ser Val Asp Tyr Asn Glu
   115 120 125
   Lys Leu Asp Phe
   130
<210> 29
   <211> 138
   <212> PRT
   <213> Human calicivirus
<400> 29
   Gly Lys Asn Lys Gly Lys Thr Lys Lys Gly Arg Gly Arg Lys Asn Asn
   1               5                   10                  15
   Tyr Asn Ala Phe Ser Arg Arg Gly Leu Ser Asp Glu Glu Tyr Glu Glu
               20                  25                  30
   Tyr Lys Lys Ile Arg Glu Glu Lys Asn Gly Asn Tyr Ser Ile Gln Glu
           35                  40                  45
   Tyr Leu Glu Asp Arg Gln Arg Tyr Glu Glu Glu Leu Ala Glu Val Gln
       50                  55                  60
   Ala Gly Gly Asp Gly Gly Ile Gly Glu Thr Glu Met Glu Ile Arg His
   65                  70                  75                  80
   Arg Val Phe Tyr Lys Ser Lys Ser Lys Lys His Gln Gln Glu Gln Arg
                   85                  90                  95
   Arg Gln Leu Gly Leu Val Thr Gly Ser Asp Ile Arg Lys Arg Lys Pro
               100                 105                 110
   Ile Asp Trp Thr Pro Pro Lys Asn Glu Trp Ala Asp Asp Asp Arg Glu
   115 120 125
   Val Asp Tyr Asn Glu Lys Ile Asn Phe Glu
   130 135
<210> 30
   <211> 124
   <212> PRT
   <213> Murine norovirus
<400> 30
   Gly Lys Lys Gly Lys Asn Lys Lys Gly Arg Gly Arg Pro Gly Val Phe
   1               5                   10                  15
   Arg Thr Arg Gly Leu Thr Asp Glu Glu Tyr Asp Glu Phe Lys Lys Arg
               20                  25                  30
   Arg Glu Ser Arg Gly Gly Lys Tyr Ser Ile Asp Asp Tyr Leu Ala Asp
           35                  40                  45
   Arg Glu Arg Glu Glu Glu Leu Leu Glu Arg Asp Glu Glu Glu Ala Ile
       50                  55                  60
   Phe Gly Asp Gly Phe Gly Leu Lys Ala Thr Arg Arg Ser Arg Lys Ala
   65                  70                  75                      80
   Glu Arg Ala Lys Leu Gly Leu Val Ser Gly Gly Asp Ile Arg Ala Arg
                   85                  90                  95
   Lys Pro Ile Asp Trp Asn Val Val Gly Pro Ser Trp Ala Asp Asp Asp
               100                 105                 110
   Arg Gln Val Asp Tyr Gly Glu Lys Ile Asn Phe Glu
           115                 120